(19)

**Europäisches Patentamt**
**European Patent Office**
**Office européen des brevets**

(11) **EP 3 772 065 A1**

(12) **EUROPEAN PATENT APPLICATION**

(43) Date of publication:
**03.02.2021 Bulletin 2021/05**

(21) Application number: **19189259.5**

(22) Date of filing: **31.07.2019**

(51) Int Cl.:
**G16B 15/30** *(2019.01)*     **G16B 15/20** *(2019.01)*
**C07K 14/705** *(2006.01)*     **C07K 14/72** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Ecole Polytechnique Federale De Lausanne (EPFL) EPFL-TTO**
**1015 Lausanne (CH)**

(72) Inventor: **Barth, Patrick**
**1025 St-Sulpice (CH)**

(74) Representative: **KATZAROV S.A.**
**European Patent Attorneys**
**12, Avenue des Morgines**
**1213 Petit-Lancy (CH)**

(54) **METHOD FOR GENERATING VARIANTS OF A PROTEIN**

(57)     The present relates to a method for generating variants of a protein based on a native protein regulated by allosteric pathway, the method comprising:
- i) providing 3D structures of the native protein;
- ii) identifying at least one pair of coupled allosteric sites within the amino acid sequence of the native protein named microswitch;
- iii) generating in silico mutations of said identified microswitch to generate a pool of variants;
- iv) computing at least one score reflecting the variation in allosteric coupling; and/or the variation in the relative stability
- v) predicting the activity of each variant compared to the native protein based on the computed score.

The invention also concern a computer implemented program to carry out said method, and a variant of a protein or an active fragment thereof, a polynucleotide.

Figure 1

EP 3 772 065 A1

SEQUENCE LISTING

<110>    ECOLE POLYTECHNIQUE FEDERALE DE LAUSANNE (EPFL)

<120>    Method for generating variants of a protein

<130>    51699/EP

<160>    32

<170>    PatentIn version 3.5

<210>    1
<211>    471
<212>    PRT
<213>    Unknown

<220>
<223>    Artificial sequences

<400>    1

Met Tyr Pro Tyr Asp Val Pro Asp Tyr Ala Tyr Pro Tyr Asp Val Pro
1               5                   10                  15

Asp Tyr Ala Tyr Pro Tyr Asp Val Pro Asp Tyr Ala Asp Asp Pro Leu
            20                  25                  30

Asn Leu Ser Trp Tyr Asp Asp Asp Leu Glu Arg Gln Asn Trp Ser Arg
            35                  40                  45

Pro Phe Asn Gly Ser Asp Gly Lys Ala Asp Arg Pro His Tyr Asn Tyr
        50                  55                  60

Tyr Ala Thr Leu Leu Thr Leu Leu Ile Ala Val Ile Val Phe Gly Asn
65                  70                  75                  80

Val Leu Val Cys Met Ala Val Ser Arg Glu Lys Ala Leu Gln Thr Thr
                85                  90                  95

Thr Asn Tyr Leu Ile Val Ser Leu Ala Val Ala Asp Leu Leu Val Ala
            100                 105                 110

Thr Leu Val Met Pro Trp Val Val Tyr Leu Glu Val Val Gly Glu Trp
            115                 120                 125

Lys Phe Ser Arg Ile His Cys Asp Ile Phe Val Thr Leu Asp Val Met
            130                 135                 140

Met Cys Thr Ala Ser Ile Leu Asn Leu Cys Ala Ile Ser Ile Asp Arg
145                 150                 155                 160

Tyr Thr Ala Val Ala Met Pro Met Leu Tyr Asn Thr Arg Tyr Ser Ser

165 170 175

Lys Arg Arg Val Thr Val Met Ile Ser Ile Val Trp Val Leu Ser Phe
180 185 190

Thr Ile Ser Cys Pro Leu Leu Phe Gly Leu Asn Asn Ala Asp Gln Asn
195 200 205

Glu Cys Ile Ile Ala Asn Pro Ala Phe Val Val Tyr Ser Ser Ile Val
210 215 220

Ser Phe Tyr Val Pro Phe Ile Val Thr Leu Leu Val Tyr Ile Lys Ile
225 230 235 240

Tyr Ile Val Leu Arg Arg Arg Arg Lys Arg Val Asn Thr Lys Arg Ser
245 250 255

Ser Arg Ala Phe Arg Ala His Leu Arg Ala Pro Leu Lys Gly Asn Cys
260 265 270

Thr His Pro Glu Asp Met Lys Leu Cys Thr Val Ile Met Lys Ser Asn
275 280 285

Gly Ser Phe Pro Val Asn Arg Arg Arg Val Glu Ala Ala Arg Arg Ala
290 295 300

Gln Glu Leu Glu Met Glu Met Leu Ser Ser Thr Ser Pro Pro Glu Arg
305 310 315 320

Thr Arg Tyr Ser Pro Ile Pro Pro Ser His His Gln Leu Thr Leu Pro
325 330 335

Asp Pro Ser His His Gly Leu His Ser Thr Pro Asp Ser Pro Ala Lys
340 345 350

Pro Glu Lys Asn Gly His Ala Lys Asp His Pro Lys Ile Ala Lys Ile
355 360 365

Phe Glu Ile Gln Thr Met Pro Asn Gly Lys Thr Arg Thr Ser Leu Lys
370 375 380

Thr Met Ser Arg Arg Lys Leu Ser Gln Gln Lys Glu Lys Lys Ala Thr
385 390 395 400

Gln Met Leu Ala Ile Val Leu Gly Val Phe Ile Ile Leu Trp Leu Pro
405 410 415

```
        Phe Phe Ile Thr His Ile Leu Asn Ile His Cys Asp Cys Asn Ile Pro
                    420             425                 430


        Pro Val Leu Tyr Ser Ala Phe Thr Trp Leu Gly Tyr Val Asn Ser Ala
                    435             440                 445


        Val Asn Pro Ile Ile Tyr Thr Thr Phe Asn Ile Glu Phe Arg Lys Ala
            450             455                 460


        Phe Leu Lys Ile Leu His Cys
        465             470



        <210>   2
        <211>   471
        <212>   PRT
        <213>   Unknown

        <220>
        <223>   Artificial sequence

        <400>   2

        Met Tyr Pro Tyr Asp Val Pro Asp Tyr Ala Tyr Pro Tyr Asp Val Pro
        1               5                   10                  15


        Asp Tyr Ala Tyr Pro Tyr Asp Val Pro Asp Tyr Ala Asp Asp Pro Leu
                    20              25                  30


        Asn Leu Ser Trp Tyr Asp Asp Asp Leu Glu Arg Gln Asn Trp Ser Arg
                    35              40                  45


        Pro Phe Asn Gly Ser Asp Gly Lys Ala Asp Arg Pro His Tyr Asn Tyr
            50              55                  60


        Tyr Ala Thr Leu Leu Thr Leu Leu Ile Ala Val Ile Val Phe Gly Asn
        65              70                  75                  80


        Val Leu Val Cys Met Ala Val Ser Arg Glu Lys Ala Leu Gln Thr Thr
                    85              90                  95


        Thr Asn Tyr Leu Ile Val Ser Leu Ala Val Ala Asp Leu Leu Val Ala
                    100             105                 110


        Thr Leu Val Met Pro Trp Val Val Tyr Leu Glu Val Val Gly Glu Trp
                    115             120                 125


        Lys Phe Ser Arg Ile His Cys Asp Ile Phe Val Thr Leu Asp Val Met
            130             135                 140


        Met Cys Thr Ala Ser Ile Leu Asn Leu Cys Ala Ile Ser Ile Asp Arg
```

```
        145                  150                    155                     160


        Tyr Thr Ala Val Ala Met Pro Met Leu Tyr Asn Thr Arg Tyr Ser Ser
                    165                  170                  175


        Lys Arg Arg Val Thr Val Met Ile Ser Ile Val Trp Val Leu Ser Phe
                    180                  185                  190


        Thr Ile Ser Cys Pro Leu Leu Phe Gly Leu Asn Asn Ala Asp Gln Asn
                    195                  200                  205


        Glu Cys Ile Ile Ala Asn Pro Ala Phe Val Val Tyr Ser Ser Ile Val
            210                  215                  220


        Ser Phe Tyr Val Pro Phe Ile Val Thr Leu Leu Val Tyr Ile Lys Ile
        225                  230                  235                  240


        Tyr Ile Val Leu Arg Arg Arg Lys Arg Val Asn Thr Lys Arg Ser
                    245                  250                  255


        Ser Arg Ala Phe Arg Ala His Leu Arg Ala Pro Leu Lys Gly Asn Cys
                    260                  265                  270


        Thr His Pro Glu Asp Met Lys Leu Cys Thr Val Ile Met Lys Ser Asn
                    275                  280                  285


        Gly Ser Phe Pro Val Asn Arg Arg Arg Val Glu Ala Ala Arg Arg Ala
            290                  295                  300


        Gln Glu Leu Glu Met Glu Met Leu Ser Ser Thr Ser Pro Pro Glu Arg
        305                  310                  315                  320


        Thr Arg Tyr Ser Pro Ile Pro Pro Ser His His Gln Leu Thr Leu Pro
                    325                  330                  335


        Asp Pro Ser His His Gly Leu His Ser Thr Pro Asp Ser Pro Ala Lys
                    340                  345                  350


        Pro Glu Lys Asn Gly His Ala Lys Asp His Pro Lys Ile Ala Lys Ile
                    355                  360                  365


        Phe Glu Ile Gln Thr Met Pro Asn Gly Lys Thr Arg Thr Ser Leu Lys
            370                  375                  380


        Thr Met Ser Arg Arg Lys Leu Ser Gln Gln Lys Glu Lys Lys Ala Thr
        385                  390                  395                  400
```

```
        Gln Met Leu Ala Ile Val Leu Gly Val Phe Ile Ile Val Trp Leu Pro
                        405             410             415

        Phe Phe Ile Thr His Ile Leu Asn Ile His Cys Asp Cys Asn Ile Pro
                        420             425             430

        Pro Val Leu Tyr Ser Ala Phe Thr Trp Leu Gly Tyr Val Asn Ser Ala
                        435             440             445

        Val Asn Pro Ile Ile Tyr Thr Thr Phe Asn Ile Glu Phe Arg Lys Ala
                        450             455             460

        Phe Leu Lys Ile Leu His Cys
        465                 470


        <210>   3
        <211>   471
        <212>   PRT
        <213>   Unknown

        <220>
        <223>   Artificial sequence

        <400>   3

        Met Tyr Pro Tyr Asp Val Pro Asp Tyr Ala Tyr Pro Tyr Asp Val Pro
        1               5               10              15

        Asp Tyr Ala Tyr Pro Tyr Asp Val Pro Asp Tyr Ala Asp Asp Pro Leu
                        20              25              30

        Asn Leu Ser Trp Tyr Asp Asp Asp Leu Glu Arg Gln Asn Trp Ser Arg
                        35              40              45

        Pro Phe Asn Gly Ser Asp Gly Lys Ala Asp Arg Pro His Tyr Asn Tyr
                        50              55              60

        Tyr Ala Thr Leu Leu Thr Leu Leu Ile Ala Val Ile Val Phe Gly Asn
        65                  70              75                  80

        Val Leu Val Cys Met Ala Val Ser Arg Glu Lys Ala Leu Gln Thr Thr
                        85              90                  95

        Thr Asn Tyr Leu Ile Val Ser Leu Ala Val Ala Asp Leu Leu Val Ala
                        100             105             110

        Thr Leu Val Met Pro Trp Val Val Tyr Leu Glu Val Val Gly Glu Trp
                        115             120             125

        Lys Phe Ser Arg Ile His Cys Asp Ile Phe Val Thr Leu Asp Val Met
```

                130                      135                        140

        Met Cys Thr Ala Ser Ile Leu Asn Leu Cys Ala Ile Ser Ile Asp Arg
        145                 150                 155                    160

        Tyr Thr Ala Val Ala Met Pro Met Leu Tyr Asn Thr Arg Tyr Ser Ser
                        165                 170                    175

        Lys Arg Arg Val Thr Val Met Ile Ser Ile Val Trp Val Leu Ser Phe
                    180                 185                    190

        Thr Ile Ser Cys Pro Leu Leu Phe Gly Leu Asn Asn Ala Asp Gln Asn
                    195                 200                    205

        Glu Cys Ile Ile Ala Asn Pro Ala Phe Val Val Tyr Ser Ser Ile Val
            210                 215                 220

        Ser Phe Tyr Val Pro Met Ile Val Thr Leu Leu Val Tyr Ile Lys Ile
        225                 230                 235                    240

        Tyr Ile Val Leu Arg Arg Arg Lys Arg Val Asn Thr Lys Arg Ser
                        245                 250                    255

        Ser Arg Ala Phe Arg Ala His Leu Arg Ala Pro Leu Lys Gly Asn Cys
                    260                 265                    270

        Thr His Pro Glu Asp Met Lys Leu Cys Thr Val Ile Met Lys Ser Asn
                    275                 280                    285

        Gly Ser Phe Pro Val Asn Arg Arg Arg Val Glu Ala Ala Arg Arg Ala
                    290                 295                    300

        Gln Glu Leu Glu Met Glu Met Leu Ser Ser Thr Ser Pro Pro Glu Arg
        305                 310                 315                    320

        Thr Arg Tyr Ser Pro Ile Pro Pro Ser His His Gln Leu Thr Leu Pro
                        325                 330                    335

        Asp Pro Ser His His Gly Leu His Ser Thr Pro Asp Ser Pro Ala Lys
                    340                 345                    350

        Pro Glu Lys Asn Gly His Ala Lys Asp His Pro Lys Ile Ala Lys Ile
                    355                 360                    365

        Phe Glu Ile Gln Thr Met Pro Asn Gly Lys Thr Arg Thr Ser Leu Lys
                370                 375                    380

```
Thr Met Ser Arg Arg Lys Leu Ser Gln Gln Lys Glu Lys Lys Ala Thr
385                 390             395                 400
```

```
Gln Met Leu Ala Ile Val Leu Gly Val Phe Ile Ile Cys Trp Leu Pro
                405             410             415
```

```
Phe Phe Ile Thr His Ile Leu Asn Ile His Cys Asp Cys Asn Ile Pro
            420             425             430
```

```
Pro Val Leu Tyr Ser Ala Phe Thr Trp Leu Gly Tyr Val Asn Ser Ala
            435             440             445
```

```
Val Asn Pro Ile Ile Tyr Thr Thr Phe Asn Ile Glu Phe Arg Lys Ala
        450             455             460
```

```
Phe Leu Lys Ile Leu His Cys
465             470
```

<210> 4
<211> 471
<212> PRT
<213> Unknown

<220>
<223> Artificial sequence

<400> 4

```
Met Tyr Pro Tyr Asp Val Pro Asp Tyr Ala Tyr Pro Tyr Asp Val Pro
1               5               10              15
```

```
Asp Tyr Ala Tyr Pro Tyr Asp Val Pro Asp Tyr Ala Asp Asp Pro Leu
            20              25              30
```

```
Asn Leu Ser Trp Tyr Asp Asp Asp Leu Glu Arg Gln Asn Trp Ser Arg
            35              40              45
```

```
Pro Phe Asn Gly Ser Asp Gly Lys Ala Asp Arg Pro His Tyr Asn Tyr
        50              55              60
```

```
Tyr Ala Thr Leu Leu Thr Leu Leu Ile Ala Val Ile Val Phe Gly Asn
65              70              75              80
```

```
Val Leu Val Cys Met Ala Val Ser Arg Glu Lys Ala Leu Gln Thr Thr
                85              90              95
```

```
Thr Asn Tyr Leu Ile Val Ser Leu Ala Val Ala Asp Leu Leu Val Ala
            100             105             110
```

```
Thr Leu Val Met Pro Trp Val Val Tyr Leu Glu Val Val Gly Glu Trp
```

                    115                          120                          125

Lys Phe Ser Arg Ile His Cys Asp Ile Phe Val Thr Leu Asp Val Met
    130                 135                 140

Met Cys Thr Ala Ser Ile Leu Asn Leu Cys Ala Ile Ser Ile Asp Arg
145                 150                 155                 160

Tyr Thr Ala Val Ala Met Pro Met Leu Tyr Asn Thr Arg Tyr Ser Ser
                165                 170                 175

Lys Arg Arg Val Thr Val Met Ile Ser Ile Val Trp Val Leu Ser Phe
                180                 185                 190

Thr Ile Ser Cys Pro Leu Leu Phe Gly Leu Asn Asn Ala Asp Gln Asn
            195                 200                 205

Glu Cys Ile Ile Ala Asn Pro Ala Phe Val Val Tyr Ser Ser Ile Val
    210                 215                 220

Ser Phe Tyr Val Pro Phe Ile Val Thr Leu Leu Val Tyr Ile Lys Ile
225                 230                 235                 240

Tyr Ile Val Leu Arg Arg Arg Arg Lys Arg Val Asn Thr Lys Arg Ser
                245                 250                 255

Ser Arg Ala Phe Arg Ala His Leu Arg Ala Pro Leu Lys Gly Asn Cys
            260                 265                 270

Thr His Pro Glu Asp Met Lys Leu Cys Thr Val Ile Met Lys Ser Asn
            275                 280                 285

Gly Ser Phe Pro Val Asn Arg Arg Arg Val Glu Ala Ala Arg Arg Ala
    290                 295                 300

Gln Glu Leu Glu Met Glu Met Leu Ser Ser Thr Ser Pro Pro Glu Arg
305                 310                 315                 320

Thr Arg Tyr Ser Pro Ile Pro Pro Ser His His Gln Leu Thr Leu Pro
                325                 330                 335

Asp Pro Ser His His Gly Leu His Ser Thr Pro Asp Ser Pro Ala Lys
            340                 345                 350

Pro Glu Lys Asn Gly His Ala Lys Asp His Pro Lys Ile Ala Lys Ile
    355                 360                 365

```
Phe Glu Ile Gln Thr Met Pro Asn Gly Lys Thr Arg Thr Ser Leu Lys
    370                 375                 380

Thr Met Ser Arg Arg Lys Leu Ser Gln Gln Lys Glu Lys Lys Ala Thr
385                 390                 395                 400

Gln Met Leu Ala Ile Val Leu Gly Val Ile Ile Ile Leu Trp Leu Pro
                405                 410                 415

Phe Phe Ile Thr His Ile Leu Asn Ile His Cys Asp Cys Asn Ile Pro
                420                 425                 430

Pro Val Leu Tyr Ser Ala Phe Thr Trp Leu Gly Tyr Val Asn Ser Ala
            435                 440                 445

Val Asn Pro Ile Ile Tyr Thr Thr Phe Asn Ile Glu Phe Arg Lys Ala
    450                 455                 460

Phe Leu Lys Ile Leu His Cys
465                 470
```

```
<210>   5
<211>   471
<212>   PRT
<213>   Unknown

<220>
<223>   Artificial sequence

<400>   5
```

```
Met Tyr Pro Tyr Asp Val Pro Asp Tyr Ala Tyr Pro Tyr Asp Val Pro
1                   5                   10                  15

Asp Tyr Ala Tyr Pro Tyr Asp Val Pro Asp Tyr Ala Asp Asp Pro Leu
                20                  25                  30

Asn Leu Ser Trp Tyr Asp Asp Asp Leu Glu Arg Gln Asn Trp Ser Arg
            35                  40                  45

Pro Phe Asn Gly Ser Asp Gly Lys Ala Asp Arg Pro His Tyr Asn Tyr
    50                  55                  60

Tyr Ala Thr Leu Leu Thr Leu Leu Ile Ala Val Ile Val Phe Gly Asn
65                  70                  75                  80

Val Leu Val Cys Met Ala Val Ser Arg Glu Lys Ala Leu Gln Thr Thr
                85                  90                  95

Thr Asn Tyr Leu Ile Val Ser Leu Ala Val Ala Asp Leu Leu Val Ala
```

                    100                      105                        110


        Thr Leu Val Met Pro Trp Val Val Tyr Leu Glu Val Val Gly Glu Trp
                    115                 120                 125


        Lys Phe Ser Arg Ile His Cys Asp Ile Phe Val Thr Leu Asp Val Met
                130                 135                 140


        Met Cys Thr Ala Ser Ile Leu Asn Leu Cys Ala Ile Ser Ile Asp Arg
        145                 150                 155                 160


        Tyr Thr Ala Val Ala Met Pro Met Leu Tyr Asn Thr Arg Tyr Ser Ser
                        165                 170                 175


        Lys Arg Arg Val Thr Val Met Ile Ser Ile Val Trp Val Leu Ser Phe
                    180                 185                 190


        Thr Ile Ser Cys Pro Leu Leu Phe Gly Leu Asn Asn Ala Asp Gln Asn
                195                 200                 205


        Glu Cys Ile Ile Ala Asn Pro Ala Phe Val Val Tyr Ser Ser Ile Val
            210                 215                 220


        Ser Phe Tyr Val Pro Phe Ile Val Thr Leu Leu Val Tyr Ile Lys Ile
        225                 230                 235                 240


        Tyr Ile Val Leu Arg Arg Arg Arg Lys Arg Val Asn Thr Lys Arg Ser
                        245                 250                 255


        Ser Arg Ala Phe Arg Ala His Leu Arg Ala Pro Leu Lys Gly Asn Cys
                    260                 265                 270


        Thr His Pro Glu Asp Met Lys Leu Cys Thr Val Ile Met Lys Ser Asn
                    275                 280                 285


        Gly Ser Phe Pro Val Asn Arg Arg Arg Val Glu Ala Ala Arg Arg Ala
                290                 295                 300


        Gln Glu Leu Glu Met Glu Met Leu Ser Ser Thr Ser Pro Pro Glu Arg
        305                 310                 315                 320


        Thr Arg Tyr Ser Pro Ile Pro Pro Ser His His Gln Leu Thr Leu Pro
                        325                 330                 335


        Asp Pro Ser His His Gly Leu His Ser Thr Pro Asp Ser Pro Ala Lys
                    340                 345                 350

```
Pro Glu Lys Asn Gly His Ala Lys Asp His Pro Lys Ile Ala Lys Ile
        355                 360                 365

Phe Glu Ile Gln Thr Met Pro Asn Gly Lys Thr Arg Thr Ser Leu Lys
        370                 375                 380

Thr Met Ser Arg Arg Lys Leu Ser Gln Gln Lys Glu Lys Lys Ala Thr
385                 390                 395                 400

Gln Met Leu Ala Ile Val Leu Gly Val Ile Ile Ile Cys Trp Leu Pro
                405                 410                 415

Phe Phe Ile Thr His Ile Leu Asn Ile His Cys Asp Cys Asn Ile Pro
                420                 425                 430

Pro Val Leu Tyr Ser Ala Phe Thr Trp Leu Gly Tyr Val Asn Ser Ala
        435                 440                 445

Val Asn Pro Ile Ile Tyr Thr Thr Phe Asn Ile Glu Phe Arg Lys Ala
        450                 455                 460

Phe Leu Lys Ile Leu His Cys
465                 470


<210>  6
<211>  471
<212>  PRT
<213>  Unknown

<220>
<223>  Artificial sequence

<400>  6

Met Tyr Pro Tyr Asp Val Pro Asp Tyr Ala Tyr Pro Tyr Asp Val Pro
1               5                   10                  15

Asp Tyr Ala Tyr Pro Tyr Asp Val Pro Asp Tyr Ala Asp Asp Pro Leu
        20                  25                  30

Asn Leu Ser Trp Tyr Asp Asp Asp Leu Glu Arg Gln Asn Trp Ser Arg
        35                  40                  45

Pro Phe Asn Gly Ser Asp Gly Lys Ala Asp Arg Pro His Tyr Asn Tyr
        50                  55                  60

Tyr Ala Thr Leu Leu Thr Leu Leu Ile Ala Val Ile Val Phe Gly Asn
65                  70                  75                  80

Val Leu Val Cys Met Ala Val Ser Arg Glu Lys Ala Leu Gln Thr Thr
```

|     |     |     | 85  |     |     |     |     | 90  |     |     |     |     | 95  |     |     |
|-----|-----|-----|-----|-----|-----|-----|-----|-----|-----|-----|-----|-----|-----|-----|-----|

Thr Asn Tyr Leu Ile Val Ser Leu Ala Val Ala Asp Leu Leu Val Ala
        100             105             110

Thr Leu Val Met Pro Trp Val Val Tyr Leu Glu Val Val Gly Glu Trp
        115             120             125

Lys Phe Ser Arg Ile His Cys Asp Ile Phe Val Thr Leu Asp Val Met
        130             135             140

Met Cys Thr Ala Ser Ile Leu Asn Leu Cys Ala Ile Ser Ile Asp Arg
145             150             155             160

Tyr Thr Ala Val Ala Met Pro Met Leu Tyr Asn Thr Arg Tyr Ser Ser
            165             170             175

Lys Arg Arg Val Thr Val Met Ile Ser Ile Val Trp Val Leu Ser Phe
        180             185             190

Thr Ile Ser Cys Pro Leu Leu Phe Gly Leu Asn Asn Ala Asp Gln Asn
        195             200             205

Glu Cys Ile Ile Ala Asn Pro Ala Phe Val Val Tyr Ser Ser Ile Val
    210             215             220

Ser Phe Tyr Val Pro Phe Ile Val Thr Leu Leu Val Tyr Ile Lys Ile
225             230             235             240

Tyr Ile Val Leu Arg Arg Arg Arg Lys Arg Val Asn Thr Lys Arg Ser
            245             250             255

Ser Arg Ala Phe Arg Ala His Leu Arg Ala Pro Leu Lys Gly Asn Cys
            260             265             270

Thr His Pro Glu Asp Met Lys Leu Cys Thr Val Ile Met Lys Ser Asn
        275             280             285

Gly Ser Phe Pro Val Asn Arg Arg Arg Val Glu Ala Ala Arg Arg Ala
        290             295             300

Gln Glu Leu Glu Met Glu Met Leu Ser Ser Thr Ser Pro Pro Glu Arg
305             310             315             320

Thr Arg Tyr Ser Pro Ile Pro Pro Ser His His Gln Leu Thr Leu Pro
            325             330             335

```
Asp Pro Ser His His Gly Leu His Ser Thr Pro Asp Ser Pro Ala Lys
            340             345             350

Pro Glu Lys Asn Gly His Ala Lys Asp His Pro Lys Ile Ala Lys Ile
            355             360             365

Phe Glu Ile Gln Thr Met Pro Asn Gly Lys Thr Arg Thr Ser Leu Lys
            370             375             380

Thr Met Ser Arg Arg Lys Leu Ser Gln Gln Lys Glu Lys Lys Ala Thr
385             390             395             400

Gln Met Leu Ala Ile Val Leu Gly Val Tyr Ile Ile Cys Trp Leu Pro
            405             410             415

Phe Phe Ile Thr His Ile Leu Asn Ile His Cys Asp Cys Asn Ile Pro
            420             425             430

Pro Val Leu Tyr Ser Ala Phe Thr Trp Leu Gly Tyr Val Asn Ser Ala
            435             440             445

Val Asn Pro Ile Ile Tyr Thr Thr Phe Asn Ile Glu Phe Arg Lys Ala
            450             455             460

Phe Leu Lys Ile Leu His Cys
465             470
```

```
<210>  7
<211>  471
<212>  PRT
<213>  Unknown

<220>
<223>  Artificial sequence

<400>  7
```

```
Met Tyr Pro Tyr Asp Val Pro Asp Tyr Ala Tyr Pro Tyr Asp Val Pro
1               5               10              15

Asp Tyr Ala Tyr Pro Tyr Asp Val Pro Asp Tyr Ala Asp Asp Pro Leu
            20              25              30

Asn Leu Ser Trp Tyr Asp Asp Asp Leu Glu Arg Gln Asn Trp Ser Arg
            35              40              45

Pro Phe Asn Gly Ser Asp Gly Lys Ala Asp Arg Pro His Tyr Asn Tyr
            50              55              60

Tyr Ala Thr Leu Leu Thr Leu Leu Ile Ala Val Ile Val Phe Gly Asn
```

32

                    65                      70                      75                      80

Val Leu Val Cys Met Ala Val Ser Arg Glu Lys Ala Leu Gln Thr Thr
                85                      90                      95

Thr Asn Tyr Leu Ile Val Ser Leu Ala Val Ala Asp Leu Leu Val Ala
            100                     105                     110

Thr Leu Val Met Pro Trp Val Val Tyr Leu Glu Val Val Gly Glu Trp
            115                     120                     125

Lys Phe Ser Arg Ile His Cys Asp Ile Phe Val Thr Leu Asp Val Met
    130                     135                     140

Met Cys Thr Ala Ser Phe Leu Asn Leu Cys Ala Ile Ser Ile Asp Arg
145                     150                     155                     160

Tyr Thr Ala Val Ala Met Pro Met Leu Tyr Asn Thr Arg Tyr Ser Ser
                165                     170                     175

Lys Arg Arg Val Thr Val Met Ile Ser Ile Val Trp Val Leu Ser Phe
            180                     185                     190

Thr Ile Ser Cys Pro Leu Leu Phe Gly Leu Asn Asn Ala Asp Gln Asn
            195                     200                     205

Glu Cys Ile Ile Ala Asn Pro Ala Phe Val Val Tyr Ser Ser Ile Val
    210                     215                     220

Ser Phe Tyr Val Pro Phe Ile Val Thr Leu Leu Val Tyr Ile Lys Ile
225                     230                     235                     240

Tyr Ile Val Leu Arg Arg Arg Arg Lys Arg Val Asn Thr Lys Arg Ser
                245                     250                     255

Ser Arg Ala Phe Arg Ala His Leu Arg Ala Pro Leu Lys Gly Asn Cys
            260                     265                     270

Thr His Pro Glu Asp Met Lys Leu Cys Thr Val Ile Met Lys Ser Asn
            275                     280                     285

Gly Ser Phe Pro Val Asn Arg Arg Arg Val Glu Ala Ala Arg Arg Ala
            290                     295                     300

Gln Glu Leu Glu Met Glu Met Leu Ser Ser Thr Ser Pro Pro Glu Arg
305                     310                     315                     320

Thr Arg Tyr Ser Pro Ile Pro Pro Ser His His Gln Leu Thr Leu Pro
            325             330             335

Asp Pro Ser His His Gly Leu His Ser Thr Pro Asp Ser Pro Ala Lys
            340             345             350

Pro Glu Lys Asn Gly His Ala Lys Asp His Pro Lys Ile Ala Lys Ile
            355             360             365

Phe Glu Ile Gln Thr Met Pro Asn Gly Lys Thr Arg Thr Ser Leu Lys
            370             375             380

Thr Met Ser Arg Arg Lys Leu Ser Gln Gln Lys Glu Lys Lys Ala Thr
385             390             395             400

Gln Met Leu Ala Ile Val Leu Gly Val Phe Ile Ile Cys Trp Leu Pro
            405             410             415

Phe Phe Ile Thr His Ile Leu Asn Ile His Cys Asp Cys Asn Ile Pro
            420             425             430

Pro Val Leu Tyr Ser Ala Phe Thr Trp Leu Gly Tyr Val Asn Ser Ala
            435             440             445

Val Asn Pro Ile Ile Tyr Thr Thr Phe Asn Ile Glu Phe Arg Lys Ala
            450             455             460

Phe Leu Lys Ile Leu His Cys
465             470

<210>   8
<211>   471
<212>   PRT
<213>   Unknown

<220>
<223>   Artificial sequence

<400>   8

Met Tyr Pro Tyr Asp Val Pro Asp Tyr Ala Tyr Pro Tyr Asp Val Pro
1               5               10              15

Asp Tyr Ala Tyr Pro Tyr Asp Val Pro Asp Tyr Ala Asp Asp Pro Leu
            20              25              30

Asn Leu Ser Trp Tyr Asp Asp Asp Leu Glu Arg Gln Asn Trp Ser Arg
            35              40              45

Pro Phe Asn Gly Ser Asp Gly Lys Ala Asp Arg Pro His Tyr Asn Tyr

```
                50                        55                        60


        Tyr Ala Thr Leu Leu Thr Leu Leu Ile Ala Val Ile Val Phe Gly Asn
        65                  70                  75                  80


        Val Leu Val Cys Met Ala Val Ser Arg Glu Lys Ala Leu Gln Thr Thr
                        85                  90                  95


        Thr Asn Tyr Leu Ile Val Ser Leu Ala Val Ala Asp Leu Leu Val Ala
                    100                 105                 110


        Thr Leu Val Met Pro Trp Val Val Tyr Leu Glu Val Val Gly Glu Trp
                    115                 120                 125


        Lys Phe Ser Arg Ile His Cys Asp Ile Phe Val Thr Leu Asp Val Met
            130                 135                 140


        Met Cys Thr Ala Ser Leu Leu Asn Leu Cys Ala Ile Ser Ile Asp Arg
        145                 150                 155                 160


        Tyr Thr Ala Val Ala Met Pro Met Leu Tyr Asn Thr Arg Tyr Ser Ser
                        165                 170                 175


        Lys Arg Arg Val Thr Val Met Ile Ser Ile Val Trp Val Leu Ser Phe
                    180                 185                 190


        Thr Ile Ser Cys Pro Leu Leu Phe Gly Leu Asn Asn Ala Asp Gln Asn
                    195                 200                 205


        Glu Cys Ile Ile Ala Asn Pro Ala Phe Val Val Tyr Ser Ser Ile Val
            210                 215                 220


        Ser Phe Tyr Val Pro Phe Ile Val Thr Leu Leu Val Tyr Ile Lys Ile
        225                 230                 235                 240


        Tyr Ile Val Leu Arg Arg Arg Arg Lys Arg Val Asn Thr Lys Arg Ser
                        245                 250                 255


        Ser Arg Ala Phe Arg Ala His Leu Arg Ala Pro Leu Lys Gly Asn Cys
                    260                 265                 270


        Thr His Pro Glu Asp Met Lys Leu Cys Thr Val Ile Met Lys Ser Asn
            275                 280                 285


        Gly Ser Phe Pro Val Asn Arg Arg Arg Val Glu Ala Ala Arg Arg Ala
            290                 295                 300
```

```
Gln Glu Leu Glu Met Glu Met Leu Ser Ser Thr Ser Pro Pro Glu Arg
305             310             315             320


Thr Arg Tyr Ser Pro Ile Pro Pro Ser His His Gln Leu Thr Leu Pro
            325             330             335


Asp Pro Ser His His Gly Leu His Ser Thr Pro Asp Ser Pro Ala Lys
            340             345             350


Pro Glu Lys Asn Gly His Ala Lys Asp His Pro Lys Ile Ala Lys Ile
        355             360             365


Phe Glu Ile Gln Thr Met Pro Asn Gly Lys Thr Arg Thr Ser Leu Lys
    370             375             380


Thr Met Ser Arg Arg Lys Leu Ser Gln Gln Lys Glu Lys Lys Ala Thr
385             390             395             400


Gln Met Leu Ala Ile Val Leu Gly Val Phe Ile Ile Cys Trp Leu Pro
            405             410             415


Phe Phe Ile Thr His Ile Leu Asn Ile His Cys Asp Cys Asn Ile Pro
            420             425             430


Pro Val Leu Tyr Ser Ala Phe Thr Trp Leu Gly Tyr Val Asn Ser Ala
            435             440             445


Val Asn Pro Ile Ile Tyr Thr Thr Phe Asn Ile Glu Phe Arg Lys Ala
        450             455             460


Phe Leu Lys Ile Leu His Cys
465             470
```

```
<210>  9
<211>  471
<212>  PRT
<213>  Unknown

<220>
<223>  Artificial sequence

<400>  9
```

```
Met Tyr Pro Tyr Asp Val Pro Asp Tyr Ala Tyr Pro Tyr Asp Val Pro
1               5               10              15


Asp Tyr Ala Tyr Pro Tyr Asp Val Pro Asp Tyr Ala Asp Asp Pro Leu
            20              25              30


Asn Leu Ser Trp Tyr Asp Asp Asp Leu Glu Arg Gln Asn Trp Ser Arg
```

|     |     | 35  |     |     |     |     | 40  |     |     |     |     | 45  |     |     |     |
|-----|-----|-----|-----|-----|-----|-----|-----|-----|-----|-----|-----|-----|-----|-----|-----|

Pro Phe Asn Gly Ser Asp Gly Lys Ala Asp Arg Pro His Tyr Asn Tyr
      50              55                60

Tyr Ala Thr Leu Leu Thr Leu Leu Ile Ala Val Ile Val Phe Gly Asn
65              70              75                  80

Val Leu Val Cys Met Ala Val Ser Arg Glu Lys Ala Leu Gln Thr Thr
              85              90                  95

Thr Asn Tyr Leu Ile Val Ser Leu Ala Val Ala Asp Leu Leu Val Ala
          100             105             110

Thr Leu Val Met Pro Trp Val Val Tyr Leu Glu Val Val Gly Glu Trp
          115             120             125

Lys Phe Ser Arg Ile His Cys Asp Ile Phe Val Thr Leu Asp Val Met
      130             135             140

Met Cys Thr Ala Ser Thr Leu Asn Leu Cys Ala Ile Ser Ile Asp Arg
145             150             155                 160

Tyr Thr Ala Val Ala Met Pro Met Leu Tyr Asn Thr Arg Tyr Ser Ser
              165             170             175

Lys Arg Arg Val Thr Val Met Ile Ser Ile Val Trp Val Leu Ser Phe
          180             185             190

Thr Ile Ser Cys Pro Leu Leu Phe Gly Leu Asn Asn Ala Asp Gln Asn
      195             200             205

Glu Cys Ile Ile Ala Asn Pro Ala Phe Val Val Tyr Ser Ser Ile Val
    210             215             220

Ser Phe Tyr Val Pro Phe Ile Val Thr Leu Leu Val Tyr Ile Lys Ile
225             230             235                 240

Tyr Ile Val Leu Arg Arg Arg Arg Lys Arg Val Asn Thr Lys Arg Ser
              245             250             255

Ser Arg Ala Phe Arg Ala His Leu Arg Ala Pro Leu Lys Gly Asn Cys
          260             265             270

Thr His Pro Glu Asp Met Lys Leu Cys Thr Val Ile Met Lys Ser Asn
          275             280             285

```
Gly Ser Phe Pro Val Asn Arg Arg Arg Val Glu Ala Ala Arg Arg Ala
    290                 295             300

Gln Glu Leu Glu Met Glu Met Leu Ser Ser Thr Ser Pro Pro Glu Arg
305                 310                 315                 320

Thr Arg Tyr Ser Pro Ile Pro Pro Ser His His Gln Leu Thr Leu Pro
            325                 330                 335

Asp Pro Ser His His Gly Leu His Ser Thr Pro Asp Ser Pro Ala Lys
            340                 345                 350

Pro Glu Lys Asn Gly His Ala Lys Asp His Pro Lys Ile Ala Lys Ile
            355                 360                 365

Phe Glu Ile Gln Thr Met Pro Asn Gly Lys Thr Arg Thr Ser Leu Lys
    370                 375                 380

Thr Met Ser Arg Arg Lys Leu Ser Gln Gln Lys Glu Lys Lys Ala Thr
385                 390                 395                 400

Gln Met Leu Ala Ile Val Leu Gly Val Phe Ile Ile Cys Trp Leu Pro
            405                 410                 415

Phe Phe Ile Thr His Ile Leu Asn Ile His Cys Asp Cys Asn Ile Pro
            420                 425                 430

Pro Val Leu Tyr Ser Ala Phe Thr Trp Leu Gly Tyr Val Asn Ser Ala
            435                 440                 445

Val Asn Pro Ile Ile Tyr Thr Thr Phe Asn Ile Glu Phe Arg Lys Ala
    450                 455                 460

Phe Leu Lys Ile Leu His Cys
465                 470


<210>  10
<211>  471
<212>  PRT
<213>  Unknown

<220>
<223>  Artificial sequence

<400>  10

Met Tyr Pro Tyr Asp Val Pro Asp Tyr Ala Tyr Pro Tyr Asp Val Pro
1                 5                 10                  15

Asp Tyr Ala Tyr Pro Tyr Asp Val Pro Asp Tyr Ala Asp Asp Pro Leu
```

|   | 20 |   |   | 25 |   |   | 30 |   |
|---|---|---|---|---|---|---|---|---|

Asn Leu Ser Trp Tyr Asp Asp Asp Leu Glu Arg Gln Asn Trp Ser Arg
     35              40              45

Pro Phe Asn Gly Ser Asp Gly Lys Ala Asp Arg Pro His Tyr Asn Tyr
     50              55              60

Tyr Ala Thr Leu Leu Thr Leu Leu Ile Ala Val Ile Val Phe Gly Asn
65               70              75              80

Val Leu Val Cys Met Ala Val Ser Arg Glu Lys Ala Leu Gln Thr Thr
          85             90              95

Thr Asn Tyr Leu Ile Val Ser Leu Ala Val Ala Asp Leu Leu Val Ala
          100           105         110

Thr Leu Val Met Pro Trp Val Val Tyr Leu Glu Val Val Gly Glu Trp
          115           120         125

Lys Phe Ser Arg Ile His Cys Asp Ile Phe Val Thr Leu Asp Val Met
          130           135         140

Met Cys Thr Ala Ser Ile Leu Asn Leu Cys Ala Ile Ser Ile Asp Arg
145               150          155              160

Tyr Thr Ala Val Ala Met Pro Met Leu Tyr Asn Thr Arg Tyr Ser Ser
          165           170         175

Lys Arg Arg Val Thr Val Met Ile Ser Ile Val Trp Val Leu Ser Phe
          180           185         190

Thr Ile Ser Cys Pro Leu Leu Phe Gly Leu Asn Asn Ala Asp Gln Asn
          195           200         205

Glu Cys Ile Ile Ala Asn Pro Ala Phe Val Val Tyr Ser Ser Ile Val
     210              215              220

Ser Phe Tyr Val Pro Phe Ile Val Thr Leu Leu Val Tyr Ile Lys Ile
225               230              235              240

Tyr Ile Val Leu Arg Arg Arg Arg Lys Arg Val Asn Thr Lys Arg Ser
          245           250         255

Ser Arg Ala Phe Arg Ala His Leu Arg Ala Pro Leu Lys Gly Asn Cys
          260           265         270

```
Thr His Pro Glu Asp Met Lys Leu Cys Thr Val Ile Met Lys Ser Asn
        275             280             285

Gly Ser Phe Pro Val Asn Arg Arg Arg Val Glu Ala Ala Arg Arg Ala
        290             295             300

Gln Glu Leu Glu Met Glu Met Leu Ser Ser Thr Ser Pro Pro Glu Arg
305             310             315             320

Thr Arg Tyr Ser Pro Ile Pro Pro Ser His His Gln Leu Thr Leu Pro
            325             330             335

Asp Pro Ser His His Gly Leu His Ser Thr Pro Asp Ser Pro Ala Lys
        340             345             350

Pro Glu Lys Asn Gly His Ala Lys Asp His Pro Lys Ile Ala Lys Ile
        355             360             365

Phe Glu Ile Gln Thr Met Pro Asn Gly Lys Thr Arg Thr Ser Leu Lys
    370             375             380

Thr Met Ser Arg Arg Lys Leu Ser Gln Gln Lys Glu Lys Lys Ala Thr
385             390             395             400

Gln Met Leu Ala Ile Val Ile Gly Val Phe Ile Ile Leu Trp Leu Pro
            405             410             415

Phe Phe Ile Thr His Ile Leu Asn Ile His Cys Asp Cys Asn Ile Pro
        420             425             430

Pro Val Leu Tyr Ser Ala Phe Thr Trp Leu Gly Tyr Val Asn Ser Ala
        435             440             445

Val Asn Pro Ile Ile Tyr Thr Thr Phe Asn Ile Glu Phe Arg Lys Ala
    450             455             460

Phe Leu Lys Ile Leu His Cys
465             470
```

```
<210>  11
<211>  471
<212>  PRT
<213>  Unknown

<220>
<223>  Artificial sequence

<400>  11
```

```
Met Tyr Pro Tyr Asp Val Pro Asp Tyr Ala Tyr Pro Tyr Asp Val Pro
```

|     |     |     | 1   |     | 5   |     |     |     | 10  |     |     |     | 15  |     |     |
| --- | --- | --- | --- | --- | --- | --- | --- | --- | --- | --- | --- | --- | --- | --- | --- |

Asp Tyr Ala Tyr Pro Tyr Asp Val Pro Asp Tyr Ala Asp Asp Pro Leu
        20              25                  30

Asn Leu Ser Trp Tyr Asp Asp Asp Leu Glu Arg Gln Asn Trp Ser Arg
        35              40                  45

Pro Phe Asn Gly Ser Asp Gly Lys Ala Asp Arg Pro His Tyr Asn Tyr
        50              55                  60

Tyr Ala Thr Leu Leu Thr Leu Leu Ile Ala Val Ile Val Phe Gly Asn
65              70              75                  80

Val Leu Val Cys Met Ala Val Ser Arg Glu Lys Ala Leu Gln Thr Thr
                85              90                  95

Thr Asn Tyr Leu Ile Val Ser Leu Ala Val Ala Asp Leu Leu Val Ala
            100             105             110

Thr Leu Val Met Pro Trp Val Val Tyr Leu Glu Val Val Gly Glu Trp
        115             120             125

Lys Phe Ser Arg Ile His Cys Asp Ile Phe Val Thr Leu Asp Val Met
        130             135             140

Met Cys Thr Ala Ser Ile Leu Asn Leu Cys Ala Ile Ser Ile Asp Arg
145             150             155             160

Tyr Thr Ala Val Ala Met Pro Met Leu Tyr Asn Thr Arg Tyr Ser Ser
                165             170             175

Lys Arg Arg Val Thr Val Met Ile Ser Ile Val Trp Val Leu Ser Phe
            180             185             190

Thr Ile Ser Cys Pro Leu Leu Phe Gly Leu Asn Asn Ala Asp Gln Asn
        195             200             205

Glu Cys Ile Ile Ala Asn Pro Ala Phe Val Val Tyr Ser Ser Ile Val
    210             215             220

Ser Phe Tyr Val Pro Phe Ile Val Thr Leu Leu Val Tyr Ile Lys Ile
225             230             235             240

Tyr Ile Val Leu Arg Arg Arg Arg Lys Arg Val Asn Thr Lys Arg Ser
            245             250             255

EP 3 772 065 A1

Ser Arg Ala Phe Arg Ala His Leu Arg Ala Pro Leu Lys Gly Asn Cys
        260             265             270

Thr His Pro Glu Asp Met Lys Leu Cys Thr Val Ile Met Lys Ser Asn
        275             280             285

Gly Ser Phe Pro Val Asn Arg Arg Arg Val Glu Ala Ala Arg Arg Ala
    290             295             300

Gln Glu Leu Glu Met Glu Met Leu Ser Ser Thr Ser Pro Pro Glu Arg
305             310             315             320

Thr Arg Tyr Ser Pro Ile Pro Pro Ser His His Gln Leu Thr Leu Pro
        325             330             335

Asp Pro Ser His His Gly Leu His Ser Thr Pro Asp Ser Pro Ala Lys
        340             345             350

Pro Glu Lys Asn Gly His Ala Lys Asp His Pro Lys Ile Ala Lys Ile
        355             360             365

Phe Glu Ile Gln Thr Met Pro Asn Gly Lys Thr Arg Thr Ser Leu Lys
        370             375             380

Thr Met Ser Arg Arg Lys Leu Ser Gln Gln Lys Glu Lys Lys Ala Thr
385             390             395             400

Gln Met Leu Ala Ile Val Ile Gly Val Ile Ile Ile Cys Trp Leu Pro
            405             410             415

Phe Phe Ile Thr His Ile Leu Asn Ile His Cys Asp Cys Asn Ile Pro
        420             425             430

Pro Val Leu Tyr Ser Ala Phe Thr Trp Leu Gly Tyr Val Asn Ser Ala
        435             440             445

Val Asn Pro Ile Ile Tyr Thr Thr Phe Asn Ile Glu Phe Arg Lys Ala
        450             455             460

Phe Leu Lys Ile Leu His Cys
465             470

<210>   12
<211>   471
<212>   PRT
<213>   Unknown

<220>
<223>   Artificial sequence

42

<400> 12

```
Met Tyr Pro Tyr Asp Val Pro Asp Tyr Ala Tyr Pro Tyr Asp Val Pro
1               5                   10                  15

Asp Tyr Ala Tyr Pro Tyr Asp Val Pro Asp Tyr Ala Asp Asp Pro Leu
            20                  25                  30

Asn Leu Ser Trp Tyr Asp Asp Asp Leu Glu Arg Gln Asn Trp Ser Arg
            35                  40                  45

Pro Phe Asn Gly Ser Asp Gly Lys Ala Asp Arg Pro His Tyr Asn Tyr
        50                  55                  60

Tyr Ala Thr Leu Leu Thr Leu Leu Ile Ala Val Ile Val Phe Gly Asn
65                  70                  75                  80

Val Leu Val Cys Met Ala Val Ser Arg Glu Lys Ala Leu Gln Thr Thr
                85                  90                  95

Thr Asn Tyr Leu Ile Val Ser Leu Ala Val Ala Asp Leu Leu Val Ala
            100                 105                 110

Thr Leu Val Met Pro Trp Val Val Tyr Leu Glu Val Val Gly Glu Trp
            115                 120                 125

Lys Phe Ser Arg Ile His Cys Asp Ile Phe Val Thr Leu Asp Val Met
    130                 135                 140

Met Cys Thr Ala Ser Ile Leu Asn Leu Cys Ala Ile Ser Ile Asp Arg
145                 150                 155                 160

Tyr Thr Ala Val Ala Met Pro Met Leu Tyr Asn Thr Arg Tyr Ser Ser
                165                 170                 175

Lys Arg Arg Val Thr Val Met Ile Ser Ile Val Trp Val Leu Ser Phe
            180                 185                 190

Thr Ile Ser Cys Pro Leu Leu Phe Gly Leu Asn Asn Ala Asp Gln Asn
            195                 200                 205

Glu Cys Ile Ile Ala Asn Pro Ala Phe Val Val Tyr Ser Ser Ile Val
    210                 215                 220

Ser Phe Tyr Val Pro Phe Ile Val Thr Leu Leu Val Tyr Ile Lys Ile
225                 230                 235                 240
```

```
Tyr Ile Val Leu Arg Arg Arg Arg Lys Arg Val Asn Thr Lys Arg Ser
              245             250             255

Ser Arg Ala Phe Arg Ala His Leu Arg Ala Pro Leu Lys Gly Asn Cys
              260             265             270

Thr His Pro Glu Asp Met Lys Leu Cys Thr Val Ile Met Lys Ser Asn
              275             280             285

Gly Ser Phe Pro Val Asn Arg Arg Arg Val Glu Ala Ala Arg Arg Ala
         290             295             300

Gln Glu Leu Glu Met Glu Met Leu Ser Ser Thr Ser Pro Pro Glu Arg
305             310             315             320

Thr Arg Tyr Ser Pro Ile Pro Pro Ser His His Gln Leu Thr Leu Pro
              325             330             335

Asp Pro Ser His His Gly Leu His Ser Thr Pro Asp Ser Pro Ala Lys
              340             345             350

Pro Glu Lys Asn Gly His Ala Lys Asp His Pro Lys Ile Ala Lys Ile
         355             360             365

Phe Glu Ile Gln Thr Met Pro Asn Gly Lys Thr Arg Thr Ser Leu Lys
    370             375             380

Thr Met Ser Arg Arg Lys Leu Ser Gln Gln Lys Glu Lys Lys Ala Thr
385             390             395             400

Gln Met Leu Ala Ile Val Ile Gly Val Phe Ile Ile Cys Trp Leu Pro
              405             410             415

Phe Phe Ile Thr His Ile Leu Asn Ile His Cys Asp Cys Asn Ile Pro
              420             425             430

Pro Val Leu Tyr Ser Ala Phe Thr Trp Leu Gly Tyr Val Asn Ser Ala
         435             440             445

Val Asn Pro Ile Ile Tyr Thr Thr Phe Asn Ile Glu Phe Arg Lys Ala
    450             455             460

Phe Leu Lys Ile Leu His Cys
465             470
```

```
<210>   13
<211>   471
<212>   PRT
```

```
<213>   Unknown

<220>
<223>   Artificial sequence

<400>   13

Met Tyr Pro Tyr Asp Val Pro Asp Tyr Ala Tyr Pro Tyr Asp Val Pro
1               5                   10                  15

Asp Tyr Ala Tyr Pro Tyr Asp Val Pro Asp Tyr Ala Asp Asp Pro Leu
            20                  25                  30

Asn Leu Ser Trp Tyr Asp Asp Asp Leu Glu Arg Gln Asn Trp Ser Arg
        35                  40                  45

Pro Phe Asn Gly Ser Asp Gly Lys Ala Asp Arg Pro His Tyr Asn Tyr
        50                  55                  60

Tyr Ala Thr Leu Leu Thr Leu Leu Ile Ala Val Ile Val Phe Gly Asn
65                  70                  75                  80

Val Leu Val Cys Met Ala Val Ser Arg Glu Lys Ala Leu Gln Thr Thr
                85                  90                  95

Thr Asn Tyr Leu Ile Val Ser Met Ala Val Ala Asp Leu Leu Val Ala
            100                 105                 110

Thr Leu Val Met Pro Trp Val Val Tyr Leu Glu Val Val Gly Glu Trp
        115                 120                 125

Lys Phe Ser Arg Ile His Cys Asp Ile Phe Val Thr Leu Asp Val Met
        130                 135                 140

Met Cys Thr Ala Ser Ile Leu Asn Leu Cys Ala Ile Ser Ile Asp Arg
145                 150                 155                 160

Tyr Thr Ala Val Ala Met Pro Met Leu Tyr Asn Thr Arg Tyr Ser Ser
                165                 170                 175

Lys Arg Arg Val Thr Val Met Ile Ser Ile Val Trp Val Leu Ser Phe
            180                 185                 190

Thr Ile Ser Cys Pro Leu Leu Phe Gly Leu Asn Asn Ala Asp Gln Asn
        195                 200                 205

Glu Cys Ile Ile Ala Asn Pro Ala Phe Val Val Tyr Ser Ser Ile Val
        210                 215                 220
```

45

```
Ser Phe Tyr Val Pro Phe Ile Val Thr Leu Leu Val Tyr Ile Lys Ile
225             230             235             240

Tyr Ile Val Leu Arg Arg Arg Arg Lys Arg Val Asn Thr Lys Arg Ser
            245             250             255

Ser Arg Ala Phe Arg Ala His Leu Arg Ala Pro Leu Lys Gly Asn Cys
            260             265             270

Thr His Pro Glu Asp Met Lys Leu Cys Thr Val Ile Met Lys Ser Asn
            275             280             285

Gly Ser Phe Pro Val Asn Arg Arg Arg Val Glu Ala Ala Arg Arg Ala
            290             295             300

Gln Glu Leu Glu Met Glu Met Leu Ser Ser Thr Ser Pro Pro Glu Arg
305             310             315             320

Thr Arg Tyr Ser Pro Ile Pro Pro Ser His His Gln Leu Thr Leu Pro
            325             330             335

Asp Pro Ser His His Gly Leu His Ser Thr Pro Asp Ser Pro Ala Lys
            340             345             350

Pro Glu Lys Asn Gly His Ala Lys Asp His Pro Lys Ile Ala Lys Ile
            355             360             365

Phe Glu Ile Gln Thr Met Pro Asn Gly Lys Thr Arg Thr Ser Leu Lys
            370             375             380

Thr Met Ser Arg Arg Lys Leu Ser Gln Gln Lys Glu Lys Lys Ala Thr
385             390             395             400

Gln Met Leu Ala Ile Val Leu Gly Val Phe Ile Ile Cys Trp Leu Pro
            405             410             415

Phe Phe Ile Thr His Ile Leu Asn Ile His Cys Asp Cys Asn Ile Pro
            420             425             430

Pro Val Leu Tyr Ser Ala Phe Thr Trp Leu Gly Tyr Val Asn Ser Ala
            435             440             445

Val Asn Pro Ile Ile Tyr Thr Thr Phe Asn Ile Glu Phe Arg Lys Ala
            450             455             460

Phe Leu Lys Ile Leu His Cys
465             470
```

EP 3 772 065 A1

```
<210>   14
<211>   471
<212>   PRT
<213>   Unknown

<220>
<223>   Artificial sequence

<400>   14

Met Tyr Pro Tyr Asp Val Pro Asp Tyr Ala Tyr Pro Tyr Asp Val Pro
1               5                   10                  15

Asp Tyr Ala Tyr Pro Tyr Asp Val Pro Asp Tyr Ala Asp Asp Pro Leu
                20                  25                  30

Asn Leu Ser Trp Tyr Asp Asp Asp Leu Glu Arg Gln Asn Trp Ser Arg
            35                  40                  45

Pro Phe Asn Gly Ser Asp Gly Lys Ala Asp Arg Pro His Tyr Asn Tyr
        50                  55                  60

Tyr Ala Thr Leu Leu Thr Leu Leu Ile Ala Val Ile Val Phe Gly Asn
65                  70                  75                  80

Val Leu Val Cys Met Ala Val Ser Arg Glu Lys Ala Leu Gln Thr Thr
                85                  90                  95

Thr Asn Tyr Leu Ile Val Ser Leu Ala Val Ala Asp Leu Leu Val Ala
            100                 105                 110

Thr Leu Val Val Pro Trp Val Val Tyr Leu Glu Val Val Gly Glu Trp
        115                 120                 125

Lys Phe Ser Arg Ile His Cys Asp Ile Phe Val Thr Leu Asp Val Met
    130                 135                 140

Met Cys Thr Ala Ser Ile Leu Asn Leu Cys Ala Ile Ser Ile Asp Arg
145                 150                 155                 160

Tyr Thr Ala Val Ala Met Pro Met Leu Tyr Asn Thr Arg Tyr Ser Ser
                165                 170                 175

Lys Arg Arg Val Thr Val Met Ile Ser Ile Val Trp Val Leu Ser Phe
            180                 185                 190

Thr Ile Ser Cys Pro Leu Leu Phe Gly Leu Asn Asn Ala Asp Gln Asn
        195                 200                 205
```

47

Glu Cys Ile Ile Ala Asn Pro Ala Phe Val Val Tyr Ser Ser Ile Val
210             215             220

Ser Phe Tyr Val Pro Phe Ile Val Thr Leu Leu Val Tyr Ile Lys Ile
225             230             235             240

Tyr Ile Val Leu Arg Arg Arg Arg Lys Arg Val Asn Thr Lys Arg Ser
            245             250             255

Ser Arg Ala Phe Arg Ala His Leu Arg Ala Pro Leu Lys Gly Asn Cys
            260             265             270

Thr His Pro Glu Asp Met Lys Leu Cys Thr Val Ile Met Lys Ser Asn
            275             280             285

Gly Ser Phe Pro Val Asn Arg Arg Arg Val Glu Ala Ala Arg Arg Ala
290             295             300

Gln Glu Leu Glu Met Glu Met Leu Ser Ser Thr Ser Pro Pro Glu Arg
305             310             315             320

Thr Arg Tyr Ser Pro Ile Pro Pro Ser His His Gln Leu Thr Leu Pro
            325             330             335

Asp Pro Ser His His Gly Leu His Ser Thr Pro Asp Ser Pro Ala Lys
            340             345             350

Pro Glu Lys Asn Gly His Ala Lys Asp His Pro Lys Ile Ala Lys Ile
            355             360             365

Phe Glu Ile Gln Thr Met Pro Asn Gly Lys Thr Arg Thr Ser Leu Lys
370             375             380

Thr Met Ser Arg Arg Lys Leu Ser Gln Gln Lys Glu Lys Lys Ala Thr
385             390             395             400

Gln Met Leu Ala Ile Val Leu Gly Val Phe Ile Ile Cys Trp Leu Pro
            405             410             415

Phe Phe Ile Thr His Ile Leu Asn Ile His Cys Asp Cys Asn Ile Pro
            420             425             430

Pro Val Leu Tyr Ser Ala Phe Thr Trp Leu Gly Tyr Val Asn Ser Ala
            435             440             445

Val Asn Pro Ile Ile Tyr Thr Thr Phe Asn Ile Glu Phe Arg Lys Ala
450             455             460

48

```
Phe Leu Lys Ile Leu His Cys
465                 470
```

```
<210>  15
<211>  471
<212>  PRT
<213>  Unknown

<220>
<223>  Artificial sequence

<400>  15
```

```
Met Tyr Pro Tyr Asp Val Pro Asp Tyr Ala Tyr Pro Tyr Asp Val Pro
1               5               10              15
```

```
Asp Tyr Ala Tyr Pro Tyr Asp Val Pro Asp Tyr Ala Asp Asp Pro Leu
            20              25              30
```

```
Asn Leu Ser Trp Tyr Asp Asp Asp Leu Glu Arg Gln Asn Trp Ser Arg
            35              40              45
```

```
Pro Phe Asn Gly Ser Asp Gly Lys Ala Asp Arg Pro His Tyr Asn Tyr
        50              55              60
```

```
Tyr Ala Thr Leu Leu Thr Leu Leu Ile Ala Val Ile Val Phe Gly Asn
65              70              75              80
```

```
Val Leu Val Cys Met Ala Val Ser Arg Glu Lys Ala Leu Gln Thr Thr
            85              90              95
```

```
Thr Asn Tyr Leu Ile Val Ser Leu Ala Val Ala Asp Leu Leu Val Ala
        100             105             110
```

```
Thr Leu Val Met Pro Trp Val Val Tyr Leu Glu Val Val Gly Glu Trp
        115             120             125
```

```
Lys Phe Ser Arg Ile His Cys Asp Ile Phe Val Thr Leu Asp Val Met
        130             135             140
```

```
Met Cys Thr Ala Ser Ile Leu Asn Leu Cys Ala Ile Ser Ile Asp Arg
145             150             155             160
```

```
Tyr Thr Ala Val Ala Met Pro Met Leu Tyr Asn Thr Arg Tyr Ser Ser
            165             170             175
```

```
Lys Arg Arg Val Thr Val Met Ile Ser Ile Val Trp Val Leu Ser Phe
            180             185             190
```

Thr Ile Ser Cys Pro Leu Leu Phe Gly Leu Asn Asn Ala Asp Gln Asn
        195                 200                 205

Glu Cys Ile Ile Ala Asn Pro Ala Phe Val Val Tyr Ser Ser Ile Val
        210                 215                 220

Ser Phe Tyr Val Pro Phe Ile Val Thr Leu Leu Val Tyr Ile Lys Ile
225                 230                 235                 240

Tyr Ile Val Leu Arg Arg Arg Arg Lys Arg Val Asn Thr Lys Arg Ser
                245                 250                 255

Ser Arg Ala Phe Arg Ala His Leu Arg Ala Pro Leu Lys Gly Asn Cys
            260                 265                 270

Thr His Pro Glu Asp Met Lys Leu Cys Thr Val Ile Met Lys Ser Asn
        275                 280                 285

Gly Ser Phe Pro Val Asn Arg Arg Arg Val Glu Ala Ala Arg Arg Ala
    290                 295                 300

Gln Glu Leu Glu Met Glu Met Leu Ser Ser Thr Ser Pro Pro Glu Arg
305                 310                 315                 320

Thr Arg Tyr Ser Pro Ile Pro Pro Ser His His Gln Leu Thr Leu Pro
                325                 330                 335

Asp Pro Ser His His Gly Leu His Ser Thr Pro Asp Ser Pro Ala Lys
            340                 345                 350

Pro Glu Lys Asn Gly His Ala Lys Asp His Pro Lys Ile Ala Lys Ile
        355                 360                 365

Phe Glu Ile Gln Thr Met Pro Asn Gly Lys Thr Arg Thr Ser Leu Lys
    370                 375                 380

Thr Met Ser Arg Arg Lys Leu Ser Gln Gln Lys Glu Lys Lys Ala Thr
385                 390                 395                 400

Gln Met Leu Ala Ile Val Leu Gly Val Phe Ile Ile Cys Trp Leu Pro
            405                 410                 415

Phe Phe Ile Ser His Ile Leu Asn Ile His Cys Asp Cys Asn Ile Pro
        420                 425                 430

Pro Val Gly Tyr Ser Ala Trp Thr Trp Leu Gly Tyr Val Asn Ser Ala
        435                 440                 445

50

```
Val Asn Pro Ile Ile Tyr Thr Thr Phe Asn Ile Glu Phe Arg Lys Ala
    450             455             460

Phe Leu Lys Ile Leu His Cys
465             470


<210>  16
<211>  471
<212>  PRT
<213>  Unknown

<220>
<223>  Artificial sequence

<400>  16

Met Tyr Pro Tyr Asp Val Pro Asp Tyr Ala Tyr Pro Tyr Asp Val Pro
1               5               10              15

Asp Tyr Ala Tyr Pro Tyr Asp Val Pro Asp Tyr Ala Asp Asp Pro Leu
            20              25              30

Asn Leu Ser Trp Tyr Asp Asp Asp Leu Glu Arg Gln Asn Trp Ser Arg
            35              40              45

Pro Phe Asn Gly Ser Asp Gly Lys Ala Asp Arg Pro His Tyr Asn Tyr
    50              55              60

Tyr Ala Thr Leu Leu Thr Leu Leu Ile Ala Val Ile Val Phe Gly Asn
65              70              75              80

Val Leu Val Cys Met Ala Val Ser Arg Glu Lys Ala Leu Gln Thr Thr
            85              90              95

Thr Asn Tyr Leu Tyr Val Ser Leu Ala Val Ala Asp Leu Leu Val Ala
            100             105             110

Thr Leu Val Met Pro Trp Val Val Tyr Leu Glu Val Val Gly Glu Trp
            115             120             125

Lys Phe Ser Arg Ile His Cys Asp Ile Phe Val Thr Leu Asp Val Met
    130             135             140

Met Cys Thr Ala Ser Ile Leu Asn Leu Cys Ala Ile Ser Ile Asp Arg
145             150             155             160

Tyr Thr Ala Val Ala Met Pro Met Leu Tyr Asn Thr Arg Tyr Ser Ser
            165             170             175
```

```
Lys Arg Arg Val Thr Val Met Ile Ser Ile Val Trp Val Leu Ser Phe
        180                 185                 190

Thr Ile Ser Cys Pro Leu Leu Phe Gly Leu Asn Asn Ala Asp Gln Asn
        195                 200                 205

Glu Cys Ile Ile Ala Asn Pro Ala Phe Val Val Tyr Ser Ser Ile Val
    210                 215                 220

Ser Phe Tyr Val Pro Leu Ile Val Ile Ile Leu Val Tyr Ile Lys Ile
225                 230                 235                 240

Tyr Ile Val Leu Arg Arg Arg Arg Lys Arg Val Asn Thr Lys Arg Ser
                245                 250                 255

Ser Arg Ala Phe Arg Ala His Leu Arg Ala Pro Leu Lys Gly Asn Cys
            260                 265                 270

Thr His Pro Glu Asp Met Lys Leu Cys Thr Val Ile Met Lys Ser Asn
        275                 280                 285

Gly Ser Phe Pro Val Asn Arg Arg Arg Val Glu Ala Ala Arg Arg Ala
    290                 295                 300

Gln Glu Leu Glu Met Glu Met Leu Ser Ser Thr Ser Pro Pro Glu Arg
305                 310                 315                 320

Thr Arg Tyr Ser Pro Ile Pro Pro Ser His His Gln Leu Thr Leu Pro
                325                 330                 335

Asp Pro Ser His His Gly Leu His Ser Thr Pro Asp Ser Pro Ala Lys
            340                 345                 350

Pro Glu Lys Asn Gly His Ala Lys Asp His Pro Lys Ile Ala Lys Ile
        355                 360                 365

Phe Glu Ile Gln Thr Met Pro Asn Gly Lys Thr Arg Thr Ser Leu Lys
    370                 375                 380

Thr Met Ser Arg Arg Lys Leu Ser Gln Gln Lys Glu Lys Lys Ala Thr
385                 390                 395                 400

Gln Leu Leu Ala Ile Tyr Leu Gly Leu Phe Leu Ile Cys Trp Leu Pro
            405                 410                 415

Phe Phe Ile Ser His Ile Leu Asn Ile His Cys Asp Cys Asn Ile Pro
        420                 425                 430
```

52

```
Pro Val Gly Tyr Ser Ala Trp Thr Trp Leu Gly Tyr Val Asn Ser Ala
        435                 440             445

Ile Asn Pro Ile Ile Phe Ala Thr Phe Asn Ile Glu Phe Arg Lys Ala
        450             455             460

Phe Leu Lys Ile Leu His Cys
465                 470


<210>  17
<211>  471
<212>  PRT
<213>  Unknown

<220>
<223>  Artificial sequence

<400>  17

Met Tyr Pro Tyr Asp Val Pro Asp Tyr Ala Tyr Pro Tyr Asp Val Pro
1               5               10              15

Asp Tyr Ala Tyr Pro Tyr Asp Val Pro Asp Tyr Ala Asp Asp Pro Leu
        20              25              30

Asn Leu Ser Trp Tyr Asp Asp Asp Leu Glu Arg Gln Asn Trp Ser Arg
        35              40              45

Pro Phe Asn Gly Ser Asp Gly Lys Ala Asp Arg Pro His Tyr Asn Tyr
    50              55              60

Tyr Ala Thr Leu Leu Thr Leu Leu Ile Ala Val Ile Val Phe Gly Asn
65              70              75              80

Val Leu Val Cys Met Ala Val Ser Arg Glu Lys Ala Leu Gln Thr Thr
            85              90              95

Thr Asn Tyr Leu Ile Val Ser Leu Ala Val Ala Asp Leu Leu Val Ala
        100             105             110

Thr Leu Val Met Pro Trp Val Val Tyr Leu Glu Val Val Gly Glu Trp
        115             120             125

Lys Phe Ser Arg Ile His Cys Asp Ile Phe Val Thr Leu Asp Val Met
    130             135             140

Met Cys Thr Ala Ser Ile Leu Asn Leu Cys Ala Ile Ser Ile Asp Arg
145             150             155             160
```

Tyr Thr Ala Val Ala Met Pro Met Leu Tyr Asn Thr Arg Tyr Ser Ser
              165              170              175

Lys Arg Arg Val Thr Val Met Ile Ser Ile Val Trp Val Leu Ser Phe
              180              185              190

Thr Ile Ser Cys Pro Leu Leu Phe Gly Leu Asn Asn Ala Asp Gln Asn
              195              200              205

Glu Cys Ile Ile Ala Asn Pro Ala Phe Val Val Tyr Ser Ser Ile Val
    210              215              220

Ser Phe Tyr Val Pro Leu Ile Val Ile Ile Leu Val Tyr Ile Lys Ile
225              230              235              240

Tyr Ile Val Leu Arg Arg Arg Arg Lys Arg Val Asn Thr Lys Arg Ser
              245              250              255

Ser Arg Ala Phe Arg Ala His Leu Arg Ala Pro Leu Lys Gly Asn Cys
              260              265              270

Thr His Pro Glu Asp Met Lys Leu Cys Thr Val Ile Met Lys Ser Asn
              275              280              285

Gly Ser Phe Pro Val Asn Arg Arg Arg Val Glu Ala Ala Arg Arg Ala
    290              295              300

Gln Glu Leu Glu Met Glu Met Leu Ser Ser Thr Ser Pro Pro Glu Arg
305              310              315              320

Thr Arg Tyr Ser Pro Ile Pro Pro Ser His His Gln Leu Thr Leu Pro
              325              330              335

Asp Pro Ser His His Gly Leu His Ser Thr Pro Asp Ser Pro Ala Lys
              340              345              350

Pro Glu Lys Asn Gly His Ala Lys Asp His Pro Lys Ile Ala Lys Ile
    355              360              365

Phe Glu Ile Gln Thr Met Pro Asn Gly Lys Thr Arg Thr Ser Leu Lys
    370              375              380

Thr Met Ser Arg Arg Lys Leu Ser Gln Gln Lys Glu Lys Lys Ala Thr
385              390              395              400

Gln Met Leu Ala Ile Val Leu Gly Val Phe Leu Ile Cys Trp Leu Pro
              405              410              415

```
Phe Phe Ile Thr His Ile Leu Asn Ile His Cys Asp Cys Asn Ile Pro
        420             425             430

Pro Val Leu Tyr Ser Ala Phe Thr Trp Leu Gly Tyr Val Asn Ser Ala
        435             440             445

Val Asn Pro Ile Ile Tyr Thr Thr Phe Asn Ile Glu Phe Arg Lys Ala
    450             455             460

Phe Leu Lys Ile Leu His Cys
465             470
```

```
<210>  18
<211>  471
<212>  PRT
<213>  Unknown

<220>
<223>  Artificial sequence

<400>  18
```

```
Met Tyr Pro Tyr Asp Val Pro Asp Tyr Ala Tyr Pro Tyr Asp Val Pro
1               5               10              15

Asp Tyr Ala Tyr Pro Tyr Asp Val Pro Asp Tyr Ala Asp Asp Pro Leu
        20              25              30

Asn Leu Ser Trp Tyr Asp Asp Asp Leu Glu Arg Gln Asn Trp Ser Arg
        35              40              45

Pro Phe Asn Gly Ser Asp Gly Lys Ala Asp Arg Pro His Tyr Asn Tyr
    50              55              60

Tyr Ala Thr Leu Leu Thr Leu Leu Ile Ala Val Ile Val Phe Gly Asn
65              70              75              80

Val Leu Val Cys Met Ala Val Ser Arg Glu Lys Ala Leu Gln Thr Thr
        85              90              95

Thr Asn Tyr Leu Ile Val Ser Leu Ala Val Ala Asp Leu Leu Val Ala
        100             105             110

Thr Leu Val Met Pro Trp Val Val Tyr Leu Glu Val Val Gly Glu Trp
        115             120             125

Lys Phe Ser Arg Ile His Cys Asp Ile Phe Val Thr Leu Asp Val Met
    130             135             140
```

```
Met Cys Thr Ala Ser Ile Leu Asn Leu Cys Ala Ile Ser Ile Asp Arg
145             150             155             160

Tyr Thr Ala Val Ala Met Pro Met Leu Tyr Asn Thr Arg Tyr Ser Ser
            165             170             175

Lys Arg Arg Val Thr Val Met Ile Ser Ile Val Trp Val Leu Ser Phe
            180             185             190

Thr Ile Ser Cys Pro Leu Leu Phe Gly Leu Asn Asn Ala Asp Gln Asn
            195             200             205

Glu Cys Ile Ile Ala Asn Pro Ala Phe Val Val Tyr Ser Ser Ile Val
    210             215             220

Ser Phe Tyr Val Pro Leu Ile Val Ile Ile Leu Val Tyr Ile Lys Ile
225             230             235             240

Tyr Ile Val Leu Arg Arg Arg Arg Lys Arg Val Asn Thr Lys Arg Ser
            245             250             255

Ser Arg Ala Phe Arg Ala His Leu Arg Ala Pro Leu Lys Gly Asn Cys
            260             265             270

Thr His Pro Glu Asp Met Lys Leu Cys Thr Val Ile Met Lys Ser Asn
            275             280             285

Gly Ser Phe Pro Val Asn Arg Arg Arg Val Glu Ala Ala Arg Arg Ala
    290             295             300

Gln Glu Leu Glu Met Glu Met Leu Ser Ser Thr Ser Pro Pro Glu Arg
305             310             315             320

Thr Arg Tyr Ser Pro Ile Pro Pro Ser His His Gln Leu Thr Leu Pro
            325             330             335

Asp Pro Ser His His Gly Leu His Ser Thr Pro Asp Ser Pro Ala Lys
            340             345             350

Pro Glu Lys Asn Gly His Ala Lys Asp His Pro Lys Ile Ala Lys Ile
            355             360             365

Phe Glu Ile Gln Thr Met Pro Asn Gly Lys Thr Arg Thr Ser Leu Lys
    370             375             380

Thr Met Ser Arg Arg Lys Leu Ser Gln Gln Lys Glu Lys Lys Ala Thr
385             390             395             400
```

56

```
Gln Leu Leu Ala Ile Tyr Leu Gly Leu Phe Leu Ile Cys Trp Leu Pro
             405                 410                 415

Phe Phe Ile Thr His Ile Leu Asn Ile His Cys Asp Cys Asn Ile Pro
             420                 425                 430

Pro Val Leu Tyr Ser Ala Phe Thr Trp Leu Gly Tyr Val Asn Ser Ala
             435                 440                 445

Ile Asn Pro Ile Ile Tyr Thr Thr Phe Asn Ile Glu Phe Arg Lys Ala
        450                 455                 460

Phe Leu Lys Ile Leu His Cys
465                 470
```

```
<210>  19
<211>  471
<212>  PRT
<213>  Unknown

<220>
<223>  Artificial sequence

<400>  19
```

```
Met Tyr Pro Tyr Asp Val Pro Asp Tyr Ala Tyr Pro Tyr Asp Val Pro
1               5                   10                  15

Asp Tyr Ala Tyr Pro Tyr Asp Val Pro Asp Tyr Ala Asp Asp Pro Leu
             20                  25                  30

Asn Leu Ser Trp Tyr Asp Asp Asp Leu Glu Arg Gln Asn Trp Ser Arg
             35                  40                  45

Pro Phe Asn Gly Ser Asp Gly Lys Ala Asp Arg Pro His Tyr Asn Tyr
        50                  55                  60

Tyr Ala Thr Leu Leu Thr Leu Leu Ile Ala Val Ile Val Phe Gly Asn
65                  70                  75                  80

Val Leu Val Cys Met Ala Val Ser Arg Glu Lys Ala Leu Gln Thr Thr
             85                  90                  95

Thr Asn Tyr Leu Tyr Val Ser Leu Ala Val Ala Asp Leu Leu Val Ala
             100                 105                 110

Thr Leu Val Met Pro Trp Val Val Tyr Leu Glu Val Val Gly Glu Trp
             115                 120                 125
```

57

```
Lys Phe Ser Arg Ile His Cys Asp Ile Phe Val Thr Leu Asp Val Met
    130                 135                 140

Met Cys Thr Ala Ser Ile Leu Asn Leu Cys Ala Ile Ser Ile Asp Arg
    145                 150                 155                 160

Tyr Thr Ala Val Ala Met Pro Met Leu Tyr Asn Thr Arg Tyr Ser Ser
                165                 170                 175

Lys Arg Arg Val Thr Val Met Ile Ser Ile Val Trp Val Leu Ser Phe
                180                 185                 190

Thr Ile Ser Cys Pro Leu Leu Phe Gly Leu Asn Asn Ala Asp Gln Asn
    195                 200                 205

Glu Cys Ile Ile Ala Asn Pro Ala Phe Val Val Tyr Ser Ser Ile Val
    210                 215                 220

Ser Phe Tyr Val Pro Leu Ile Val Ile Ile Leu Val Tyr Ile Lys Ile
225                 230                 235                 240

Tyr Ile Val Leu Arg Arg Arg Arg Lys Arg Val Asn Thr Lys Arg Ser
                245                 250                 255

Ser Arg Ala Phe Arg Ala His Leu Arg Ala Pro Leu Lys Gly Asn Cys
                260                 265                 270

Thr His Pro Glu Asp Met Lys Leu Cys Thr Val Ile Met Lys Ser Asn
    275                 280                 285

Gly Ser Phe Pro Val Asn Arg Arg Arg Val Glu Ala Ala Arg Arg Ala
    290                 295                 300

Gln Glu Leu Glu Met Glu Met Leu Ser Ser Thr Ser Pro Pro Glu Arg
305                 310                 315                 320

Thr Arg Tyr Ser Pro Ile Pro Pro Ser His His Gln Leu Thr Leu Pro
                325                 330                 335

Asp Pro Ser His His Gly Leu His Ser Thr Pro Asp Ser Pro Ala Lys
                340                 345                 350

Pro Glu Lys Asn Gly His Ala Lys Asp His Pro Lys Ile Ala Lys Ile
    355                 360                 365

Phe Glu Ile Gln Thr Met Pro Asn Gly Lys Thr Arg Thr Ser Leu Lys
    370                 375                 380
```

```
Thr Met Ser Arg Arg Lys Leu Ser Gln Gln Lys Glu Lys Lys Ala Thr
385             390             395             400

Gln Leu Leu Ala Ile Tyr Leu Gly Leu Phe Leu Ile Cys Trp Leu Pro
            405             410             415

Phe Phe Ile Thr His Ile Leu Asn Ile His Cys Asp Cys Asn Ile Pro
            420             425             430

Pro Val Leu Tyr Ser Ala Phe Thr Trp Leu Gly Tyr Val Asn Ser Ala
        435             440             445

Ile Asn Pro Ile Ile Phe Ala Thr Phe Asn Ile Glu Phe Arg Lys Ala
    450             455             460

Phe Leu Lys Ile Leu His Cys
465             470


<210>   20
<211>   471
<212>   PRT
<213>   Unknown

<220>
<223>   Artificial sequence

<400>   20

Met Tyr Pro Tyr Asp Val Pro Asp Tyr Ala Tyr Pro Tyr Asp Val Pro
1               5               10              15

Asp Tyr Ala Tyr Pro Tyr Asp Val Pro Asp Tyr Ala Asp Asp Pro Leu
            20              25              30

Asn Leu Ser Trp Tyr Asp Asp Asp Leu Glu Arg Gln Asn Trp Ser Arg
        35              40              45

Pro Phe Asn Gly Ser Asp Gly Lys Ala Asp Arg Pro His Tyr Asn Tyr
    50              55              60

Tyr Ala Thr Leu Leu Thr Leu Leu Ile Ala Val Ile Val Phe Gly Asn
65              70              75              80

Val Leu Val Cys Met Ala Val Ser Arg Glu Lys Ala Leu Gln Thr Thr
            85              90                  95

Thr Asn Tyr Leu Ile Val Ser Leu Ala Val Ala Asp Leu Leu Val Ala
        100             105             110
```

Thr Leu Val Met Pro Trp Val Val Tyr Leu Glu Val Val Gly Glu Trp
115 120 125

Lys Phe Ser Arg Ile His Cys Asp Ile Phe Val Thr Leu Asp Val Met
130 135 140

Met Cys Thr Ala Ser Ile Leu Asn Leu Cys Ala Ile Ser Ile Asp Arg
145 150 155 160

Tyr Thr Ala Val Ala Met Pro Met Leu Tyr Asn Thr Arg Tyr Ser Ser
165 170 175

Lys Arg Arg Val Thr Val Met Ile Ser Ile Val Trp Val Leu Ser Phe
180 185 190

Thr Ile Ser Cys Pro Leu Leu Phe Gly Leu Asn Asn Ala Asp Gln Asn
195 200 205

Glu Cys Ile Ile Ala Asn Pro Ala Phe Val Val Tyr Ser Ser Ile Val
210 215 220

Ser Phe Tyr Val Pro Phe Ile Val Ile Leu Leu Val Tyr Ile Lys Ile
225 230 235 240

Tyr Ile Val Leu Arg Arg Arg Arg Lys Arg Val Asn Thr Lys Arg Ser
245 250 255

Ser Arg Ala Phe Arg Ala His Leu Arg Ala Pro Leu Lys Gly Asn Cys
260 265 270

Thr His Pro Glu Asp Met Lys Leu Cys Thr Val Ile Met Lys Ser Asn
275 280 285

Gly Ser Phe Pro Val Asn Arg Arg Arg Val Glu Ala Ala Arg Arg Ala
290 295 300

Gln Glu Leu Glu Met Glu Met Leu Ser Ser Thr Ser Pro Pro Glu Arg
305 310 315 320

Thr Arg Tyr Ser Pro Ile Pro Pro Ser His His Gln Leu Thr Leu Pro
325 330 335

Asp Pro Ser His His Gly Leu His Ser Thr Pro Asp Ser Pro Ala Lys
340 345 350

Pro Glu Lys Asn Gly His Ala Lys Asp His Pro Lys Ile Ala Lys Ile
355 360 365

60

```
Phe Glu Ile Gln Thr Met Pro Asn Gly Lys Thr Arg Thr Ser Leu Lys
    370             375             380

Thr Met Ser Arg Arg Lys Leu Ser Gln Gln Lys Glu Lys Lys Ala Thr
385             390             395                 400

Gln Leu Leu Ala Ile Tyr Leu Gly Leu Phe Ile Ile Cys Trp Leu Pro
                405             410             415

Phe Phe Ile Thr His Ile Leu Asn Ile His Cys Asp Cys Asn Ile Pro
            420             425             430

Pro Val Leu Tyr Ser Ala Phe Thr Trp Leu Gly Tyr Val Asn Ser Ala
        435             440             445

Ile Asn Pro Ile Ile Tyr Thr Thr Phe Asn Ile Glu Phe Arg Lys Ala
    450             455             460

Phe Leu Lys Ile Leu His Cys
465             470


<210>   21
<211>   471
<212>   PRT
<213>   Unknown

<220>
<223>   Artificial sequence

<400>   21

Met Tyr Pro Tyr Asp Val Pro Asp Tyr Ala Tyr Pro Tyr Asp Val Pro
1               5               10              15

Asp Tyr Ala Tyr Pro Tyr Asp Val Pro Asp Tyr Ala Asp Asp Pro Leu
            20              25              30

Asn Leu Ser Trp Tyr Asp Asp Asp Leu Glu Arg Gln Asn Trp Ser Arg
            35              40              45

Pro Phe Asn Gly Ser Asp Gly Lys Ala Asp Arg Pro His Tyr Asn Tyr
    50              55              60

Tyr Ala Thr Leu Leu Thr Leu Leu Ile Ala Val Ile Val Phe Gly Asn
65              70              75              80

Val Leu Val Cys Met Ala Val Ser Arg Glu Lys Ala Leu Gln Thr Thr
            85              90                  95
```

```
Thr Asn Tyr Leu Tyr Val Ser Leu Ala Val Ala Asp Leu Leu Val Ala
        100         105         110

Thr Leu Val Met Pro Trp Val Val Tyr Leu Glu Val Val Gly Glu Trp
        115         120         125

Lys Phe Ser Arg Ile His Cys Asp Ile Phe Val Thr Leu Asp Val Met
        130         135         140

Met Cys Thr Ala Ser Ile Leu Asn Leu Cys Ala Ile Ser Ile Asp Arg
145         150         155         160

Tyr Thr Ala Val Ala Met Pro Met Leu Tyr Asn Thr Arg Tyr Ser Ser
            165         170         175

Lys Arg Arg Val Thr Val Met Ile Ser Ile Val Trp Val Leu Ser Phe
        180         185         190

Thr Ile Ser Cys Pro Leu Leu Phe Gly Leu Asn Asn Ala Asp Gln Asn
        195         200         205

Glu Cys Ile Ile Ala Asn Pro Ala Phe Val Val Tyr Ser Ser Ile Val
    210         215         220

Ser Phe Tyr Val Pro Phe Ile Val Thr Leu Leu Val Tyr Ile Lys Ile
225         230         235         240

Tyr Ile Val Leu Arg Arg Arg Arg Lys Arg Val Asn Thr Lys Arg Ser
            245         250         255

Ser Arg Ala Phe Arg Ala His Leu Arg Ala Pro Leu Lys Gly Asn Cys
            260         265         270

Thr His Pro Glu Asp Met Lys Leu Cys Thr Val Ile Met Lys Ser Asn
        275         280         285

Gly Ser Phe Pro Val Asn Arg Arg Arg Val Glu Ala Ala Arg Arg Ala
        290         295         300

Gln Glu Leu Glu Met Glu Met Leu Ser Ser Thr Ser Pro Pro Glu Arg
305         310         315         320

Thr Arg Tyr Ser Pro Ile Pro Pro Ser His His Gln Leu Thr Leu Pro
            325         330         335

Asp Pro Ser His His Gly Leu His Ser Thr Pro Asp Ser Pro Ala Lys
        340         345         350
```

```
Pro Glu Lys Asn Gly His Ala Lys Asp His Pro Lys Ile Ala Lys Ile
    355                 360             365

Phe Glu Ile Gln Thr Met Pro Asn Gly Lys Thr Arg Thr Ser Leu Lys
    370             375             380

Thr Met Ser Arg Arg Lys Leu Ser Gln Gln Lys Glu Lys Lys Ala Thr
385             390             395                     400

Gln Met Leu Ala Ile Val Leu Gly Val Phe Ile Ile Cys Trp Leu Pro
            405             410             415

Phe Phe Ile Thr His Ile Leu Asn Ile His Cys Asp Cys Asn Ile Pro
        420             425             430

Pro Val Leu Tyr Ser Ala Phe Thr Trp Leu Gly Tyr Val Asn Ser Ala
    435             440             445

Val Asn Pro Ile Ile Phe Ala Thr Phe Asn Ile Glu Phe Arg Lys Ala
    450             455             460

Phe Leu Lys Ile Leu His Cys
465             470
```

<210> 22
<211> 471
<212> PRT
<213> Unknown

<220>
<223> Artificial sequence

<400> 22

```
Met Tyr Pro Tyr Asp Val Pro Asp Tyr Ala Tyr Pro Tyr Asp Val Pro
1           5               10              15

Asp Tyr Ala Tyr Pro Tyr Asp Val Pro Asp Tyr Ala Asp Asp Pro Leu
        20              25              30

Asn Leu Ser Trp Tyr Asp Asp Asp Leu Glu Arg Gln Asn Trp Ser Arg
        35              40              45

Pro Phe Asn Gly Ser Asp Gly Lys Ala Asp Arg Pro His Tyr Asn Tyr
    50              55              60

Tyr Ala Thr Leu Leu Thr Leu Leu Ile Ala Val Ile Val Phe Gly Asn
65              70              75              80
```

```
Val Leu Val Cys Met Ala Val Ser Arg Glu Lys Ala Leu Gln Thr Thr
            85              90                  95

Thr Asn Tyr Leu Ile Val Ser Leu Ala Val Ala Asp Leu Leu Val Ala
            100             105                 110

Thr Leu Val Met Pro Trp Val Val Tyr Leu Glu Val Val Gly Glu Trp
            115             120                 125

Lys Phe Ser Arg Ile His Cys Asp Ile Phe Val Thr Leu Asp Val Met
            130             135                 140

Met Cys Thr Ala Ser Ile Leu Asn Leu Cys Ala Ile Ser Ile Asp Arg
145             150             155                 160

Tyr Thr Ala Val Ala Met Pro Met Leu Tyr Asn Thr Arg Tyr Ser Ser
            165             170                 175

Lys Arg Arg Val Thr Val Met Ile Ser Ile Val Trp Val Leu Ser Phe
            180             185                 190

Thr Ile Ser Cys Pro Leu Leu Phe Gly Leu Asn Asn Ala Asp Gln Asn
            195             200                 205

Glu Cys Ile Ile Ala Asn Pro Ala Phe Val Val Tyr Ser Ser Ile Val
210             215             220

Ser Phe Tyr Val Pro Phe Ile Val Ile Leu Leu Val Tyr Ile Lys Ile
225             230             235                 240

Tyr Ile Val Leu Arg Arg Arg Arg Lys Arg Val Asn Thr Lys Arg Ser
            245             250                 255

Ser Arg Ala Phe Arg Ala His Leu Arg Ala Pro Leu Lys Gly Asn Cys
            260             265                 270

Thr His Pro Glu Asp Met Lys Leu Cys Thr Val Ile Met Lys Ser Asn
            275             280                 285

Gly Ser Phe Pro Val Asn Arg Arg Arg Val Glu Ala Ala Arg Arg Ala
            290             295                 300

Gln Glu Leu Glu Met Glu Met Leu Ser Ser Thr Ser Pro Pro Glu Arg
305             310             315                 320

Thr Arg Tyr Ser Pro Ile Pro Pro Ser His His Gln Leu Thr Leu Pro
            325             330                 335
```

64

```
Asp Pro Ser His His Gly Leu His Ser Thr Pro Asp Ser Pro Ala Lys
        340                 345                 350

Pro Glu Lys Asn Gly His Ala Lys Asp His Pro Lys Ile Ala Lys Ile
        355                 360                 365

Phe Glu Ile Gln Thr Met Pro Asn Gly Lys Thr Arg Thr Ser Leu Lys
        370                 375                 380

Thr Met Ser Arg Arg Lys Leu Ser Gln Gln Lys Glu Lys Lys Ala Thr
385                 390                 395                 400

Gln Met Leu Ala Ile Val Leu Gly Val Phe Ile Ile Cys Trp Leu Pro
                405                 410                 415

Phe Phe Ile Thr His Ile Leu Asn Ile His Cys Asp Cys Asn Ile Pro
            420                 425                 430

Pro Val Leu Tyr Ser Ala Phe Thr Trp Leu Gly Tyr Val Asn Ser Ala
        435                 440                 445

Val Asn Pro Ile Ile Tyr Thr Thr Phe Asn Ile Glu Phe Arg Lys Ala
        450                 455                 460

Phe Leu Lys Ile Leu His Cys
465                 470
```

```
<210>   23
<211>   471
<212>   PRT
<213>   Unknown

<220>
<223>   Artificial sequence

<400>   23

Met Tyr Pro Tyr Asp Val Pro Asp Tyr Ala Tyr Pro Tyr Asp Val Pro
1                   5                   10                  15

Asp Tyr Ala Tyr Pro Tyr Asp Val Pro Asp Tyr Ala Asp Asp Pro Leu
            20                  25                  30

Asn Leu Ser Trp Tyr Asp Asp Asp Leu Glu Arg Gln Asn Trp Ser Arg
            35                  40                  45

Pro Phe Asn Gly Ser Asp Gly Lys Ala Asp Arg Pro His Tyr Asn Tyr
        50                  55                  60
```

```
Tyr Ala Thr Leu Leu Thr Leu Leu Ile Ala Val Ile Val Phe Gly Asn
65              70              75              80

Val Leu Val Cys Met Ala Val Ser Arg Glu Lys Ala Leu Gln Thr Thr
                85              90              95

Thr Asn Tyr Leu Ile Val Ser Leu Ala Val Ala Asp Leu Leu Val Ala
            100             105             110

Thr Leu Val Met Pro Trp Val Val Tyr Leu Glu Val Val Gly Glu Trp
            115             120             125

Lys Phe Ser Arg Ile His Cys Asp Ile Phe Val Thr Leu Asp Val Met
    130             135             140

Met Cys Thr Ala Ser Ile Leu Asn Leu Cys Ala Ile Ser Ile Asp Arg
145             150             155             160

Tyr Thr Ala Val Ala Met Pro Met Leu Tyr Asn Thr Arg Tyr Ser Ser
            165             170             175

Lys Arg Arg Val Thr Val Met Ile Ser Ile Val Trp Val Leu Ser Phe
            180             185             190

Thr Ile Ser Cys Pro Leu Leu Phe Gly Leu Asn Asn Ala Asp Gln Asn
    195             200             205

Glu Cys Ile Ile Ala Asn Pro Ala Phe Val Val Tyr Ser Ser Ile Val
    210             215             220

Ser Phe Tyr Val Pro Phe Ile Val Leu Leu Leu Val Tyr Ile Lys Ile
225             230             235             240

Tyr Ile Val Leu Arg Arg Arg Arg Lys Arg Val Asn Thr Lys Arg Ser
            245             250             255

Ser Arg Ala Phe Arg Ala His Leu Arg Ala Pro Leu Lys Gly Asn Cys
            260             265             270

Thr His Pro Glu Asp Met Lys Leu Cys Thr Val Ile Met Lys Ser Asn
    275             280             285

Gly Ser Phe Pro Val Asn Arg Arg Arg Val Glu Ala Ala Arg Arg Ala
    290             295             300

Gln Glu Leu Glu Met Glu Met Leu Ser Ser Thr Ser Pro Pro Glu Arg
305             310             315             320
```

66

Thr Arg Tyr Ser Pro Ile Pro Pro Ser His His Gln Leu Thr Leu Pro
            325             330                 335

Asp Pro Ser His His Gly Leu His Ser Thr Pro Asp Ser Pro Ala Lys
            340             345                 350

Pro Glu Lys Asn Gly His Ala Lys Asp His Pro Lys Ile Ala Lys Ile
            355             360                 365

Phe Glu Ile Gln Thr Met Pro Asn Gly Lys Thr Arg Thr Ser Leu Lys
    370             375             380

Thr Met Ser Arg Arg Lys Leu Ser Gln Gln Lys Glu Lys Lys Ala Thr
385             390                 395                 400

Gln Met Leu Ala Ile Val Leu Gly Val Phe Ile Ile Cys Trp Leu Pro
            405             410                 415

Phe Phe Ile Thr His Ile Leu Asn Ile His Cys Asp Cys Asn Ile Pro
            420             425                 430

Pro Val Leu Tyr Ser Ala Phe Thr Trp Leu Gly Tyr Val Asn Ser Ala
            435             440                 445

Val Asn Pro Ile Ile Tyr Thr Thr Phe Asn Ile Glu Phe Arg Lys Ala
    450             455             460

Phe Leu Lys Ile Leu His Cys
465             470

<210> 24
<211> 471
<212> PRT
<213> Unknown

<220>
<223> Artificial sequence

<400> 24

Met Tyr Pro Tyr Asp Val Pro Asp Tyr Ala Tyr Pro Tyr Asp Val Pro
1               5               10              15

Asp Tyr Ala Tyr Pro Tyr Asp Val Pro Asp Tyr Ala Asp Asp Pro Leu
            20              25              30

Asn Leu Ser Trp Tyr Asp Asp Asp Leu Glu Arg Gln Asn Trp Ser Arg
            35              40              45

Pro Phe Asn Gly Ser Asp Gly Lys Ala Asp Arg Pro His Tyr Asn Tyr
    50              55              60

Tyr Ala Thr Leu Leu Thr Leu Leu Ile Ala Val Ile Val Phe Gly Asn
65              70              75                      80

Val Leu Val Cys Met Ala Val Ser Arg Glu Lys Ala Leu Gln Thr Thr
            85              90                      95

Thr Asn Tyr Leu Ile Val Ser Leu Ala Val Ala Asp Leu Leu Val Ala
        100             105             110

Thr Leu Val Met Pro Trp Val Val Tyr Leu Glu Val Val Gly Glu Trp
        115             120             125

Lys Phe Ser Arg Ile His Cys Asp Ile Phe Val Thr Leu Asp Val Met
    130             135             140

Met Cys Thr Ala Ser Ile Leu Asn Leu Cys Ala Ile Ser Ile Asp Arg
145             150             155             160

Tyr Thr Ala Val Ala Met Pro Met Leu Tyr Asn Thr Arg Tyr Ser Ser
            165             170             175

Lys Arg Arg Val Thr Val Met Ile Ser Ile Val Trp Val Leu Ser Phe
        180             185             190

Thr Ile Ser Cys Pro Leu Leu Phe Gly Leu Asn Asn Ala Asp Gln Asn
        195             200             205

Glu Cys Ile Ile Ala Asn Pro Ala Phe Val Val Tyr Ser Ser Ile Val
    210             215             220

Ser Phe Tyr Val Pro Phe Ile Val Met Leu Leu Val Tyr Ile Lys Ile
225             230             235             240

Tyr Ile Val Leu Arg Arg Arg Lys Arg Val Asn Thr Lys Arg Ser
            245             250             255

Ser Arg Ala Phe Arg Ala His Leu Arg Ala Pro Leu Lys Gly Asn Cys
            260             265             270

Thr His Pro Glu Asp Met Lys Leu Cys Thr Val Ile Met Lys Ser Asn
        275             280             285

Gly Ser Phe Pro Val Asn Arg Arg Arg Val Glu Ala Ala Arg Arg Ala
        290             295             300

```
Gln Glu Leu Glu Met Glu Met Leu Ser Ser Thr Ser Pro Pro Glu Arg
305             310             315             320

Thr Arg Tyr Ser Pro Ile Pro Pro Ser His His Gln Leu Thr Leu Pro
            325             330             335

Asp Pro Ser His His Gly Leu His Ser Thr Pro Asp Ser Pro Ala Lys
        340             345             350

Pro Glu Lys Asn Gly His Ala Lys Asp His Pro Lys Ile Ala Lys Ile
    355             360             365

Phe Glu Ile Gln Thr Met Pro Asn Gly Lys Thr Arg Thr Ser Leu Lys
370             375             380

Thr Met Ser Arg Arg Lys Leu Ser Gln Gln Lys Glu Lys Lys Ala Thr
385             390             395             400

Gln Met Leu Ala Ile Val Leu Gly Val Phe Ile Ile Leu Trp Leu Pro
            405             410             415

Phe Phe Ile Thr His Ile Leu Asn Ile His Cys Asp Cys Asn Ile Pro
            420             425             430

Pro Val Leu Tyr Ser Ala Phe Thr Trp Leu Gly Tyr Val Asn Ser Ala
        435             440             445

Val Asn Pro Ile Ile Tyr Thr Thr Phe Asn Ile Glu Phe Arg Lys Ala
    450             455             460

Phe Leu Lys Ile Leu His Cys
465             470
```

```
<210>   25
<211>   471
<212>   PRT
<213>   Unknown

<220>
<223>   Artificial sequence

<400>   25
```

```
Met Tyr Pro Tyr Asp Val Pro Asp Tyr Ala Tyr Pro Tyr Asp Val Pro
1               5               10              15

Asp Tyr Ala Tyr Pro Tyr Asp Val Pro Asp Tyr Ala Asp Asp Pro Leu
            20              25              30
```

Asn Leu Ser Trp Tyr Asp Asp Asp Leu Glu Arg Gln Asn Trp Ser Arg
        35              40              45

Pro Phe Asn Gly Ser Asp Gly Lys Ala Asp Arg Pro His Tyr Asn Tyr
        50              55              60

Tyr Ala Thr Leu Leu Thr Leu Leu Ile Ala Val Ile Val Phe Gly Asn
65              70              75              80

Val Leu Val Cys Met Ala Val Ser Arg Glu Lys Ala Leu Gln Thr Thr
            85              90              95

Thr Asn Tyr Leu Ile Val Ser Leu Ala Val Ala Asp Leu Leu Val Ala
        100             105             110

Thr Leu Val Met Pro Trp Val Val Tyr Leu Glu Val Val Gly Glu Trp
        115             120             125

Lys Phe Ser Arg Ile His Cys Asp Ile Phe Val Thr Leu Asp Val Met
        130             135             140

Met Cys Thr Ala Ser Ile Leu Asn Leu Cys Ala Ile Ser Ile Asp Arg
145             150             155             160

Tyr Thr Ala Val Ala Met Pro Met Leu Tyr Asn Thr Arg Tyr Ser Ser
            165             170             175

Lys Arg Arg Val Thr Val Met Ile Ser Ile Val Trp Val Leu Ser Phe
            180             185             190

Thr Ile Ser Cys Pro Leu Leu Phe Gly Leu Asn Asn Ala Asp Gln Asn
        195             200             205

Glu Cys Ile Ile Ala Asn Pro Ala Phe Val Val Tyr Ser Ser Ile Val
        210             215             220

Ser Phe Tyr Val Pro Phe Ile Val Met Leu Leu Val Tyr Ile Lys Ile
225             230             235             240

Tyr Ile Val Leu Arg Arg Arg Arg Lys Arg Val Asn Thr Lys Arg Ser
            245             250             255

Ser Arg Ala Phe Arg Ala His Leu Arg Ala Pro Leu Lys Gly Asn Cys
            260             265             270

Thr His Pro Glu Asp Met Lys Leu Cys Thr Val Ile Met Lys Ser Asn
        275             280             285

70

```
Gly Ser Phe Pro Val Asn Arg Arg Arg Val Glu Ala Ala Arg Arg Ala
    290                 295                 300

Gln Glu Leu Glu Met Glu Met Leu Ser Ser Thr Ser Pro Pro Glu Arg
305                 310                 315                 320

Thr Arg Tyr Ser Pro Ile Pro Pro Ser His His Gln Leu Thr Leu Pro
                325                 330                 335

Asp Pro Ser His His Gly Leu His Ser Thr Pro Asp Ser Pro Ala Lys
                340                 345                 350

Pro Glu Lys Asn Gly His Ala Lys Asp His Pro Lys Ile Ala Lys Ile
            355                 360                 365

Phe Glu Ile Gln Thr Met Pro Asn Gly Lys Thr Arg Thr Ser Leu Lys
    370                 375                 380

Thr Met Ser Arg Arg Lys Leu Ser Gln Gln Lys Glu Lys Lys Ala Thr
385                 390                 395                 400

Gln Met Leu Ala Ile Val Leu Gly Val Phe Ile Ile Val Trp Leu Pro
                405                 410                 415

Phe Phe Ile Thr His Ile Leu Asn Ile His Cys Asp Cys Asn Ile Pro
                420                 425                 430

Pro Val Leu Tyr Ser Ala Phe Thr Trp Leu Gly Tyr Val Asn Ser Ala
            435                 440                 445

Val Asn Pro Ile Ile Tyr Thr Thr Phe Asn Ile Glu Phe Arg Lys Ala
    450                 455                 460

Phe Leu Lys Ile Leu His Cys
465                 470
```

```
<210>   26
<211>   471
<212>   PRT
<213>   Unknown

<220>
<223>   Artificial sequence

<400>   26
```

```
Met Tyr Pro Tyr Asp Val Pro Asp Tyr Ala Tyr Pro Tyr Asp Val Pro
1               5                   10                  15
```

```
Asp Tyr Ala Tyr Pro Tyr Asp Val Pro Asp Tyr Ala Asp Asp Pro Leu
            20              25              30

Asn Leu Ser Trp Tyr Asp Asp Asp Leu Glu Arg Gln Asn Trp Ser Arg
            35              40              45

Pro Phe Asn Gly Ser Asp Gly Lys Ala Asp Arg Pro His Tyr Asn Tyr
        50              55              60

Tyr Ala Thr Leu Leu Thr Leu Leu Ile Ala Val Ile Val Phe Gly Asn
65              70              75              80

Val Leu Val Cys Met Ala Val Ser Arg Glu Lys Ala Leu Gln Thr Thr
            85              90              95

Thr Asn Tyr Leu Ile Val Ser Leu Ala Val Ala Asp Leu Leu Val Ala
            100             105             110

Thr Leu Val Met Pro Trp Val Val Tyr Leu Glu Val Val Gly Glu Trp
        115             120             125

Lys Phe Ser Arg Ile His Cys Asp Ile Phe Val Thr Leu Asp Val Met
    130             135             140

Met Cys Thr Ala Ser Ile Leu Asn Leu Cys Ala Ile Ser Ile Asp Arg
145             150             155             160

Tyr Thr Ala Val Ala Met Pro Met Leu Tyr Asn Thr Arg Tyr Ser Ser
            165             170             175

Lys Arg Arg Val Thr Val Met Ile Ser Ile Val Trp Val Leu Ser Phe
        180             185             190

Thr Ile Ser Cys Pro Leu Leu Phe Gly Leu Asn Asn Ala Asp Gln Asn
    195             200             205

Glu Cys Ile Ile Ala Asn Pro Ala Phe Val Val Tyr Ser Ser Ile Val
210             215             220

Ser Phe Tyr Val Pro Phe Ile Val Met Leu Leu Val Tyr Ile Lys Ile
225             230             235             240

Tyr Ile Val Leu Arg Arg Arg Lys Arg Val Asn Thr Lys Arg Ser
            245             250             255

Ser Arg Ala Phe Arg Ala His Leu Arg Ala Pro Leu Lys Gly Asn Cys
        260             265             270
```

72

```
Thr His Pro Glu Asp Met Lys Leu Cys Thr Val Ile Met Lys Ser Asn
    275                 280                 285

Gly Ser Phe Pro Val Asn Arg Arg Arg Val Glu Ala Ala Arg Arg Ala
    290                 295                 300

Gln Glu Leu Glu Met Glu Met Leu Ser Ser Thr Ser Pro Pro Glu Arg
305                 310                 315                 320

Thr Arg Tyr Ser Pro Ile Pro Pro Ser His His Gln Leu Thr Leu Pro
                325                 330                 335

Asp Pro Ser His His Gly Leu His Ser Thr Pro Asp Ser Pro Ala Lys
                340                 345                 350

Pro Glu Lys Asn Gly His Ala Lys Asp His Pro Lys Ile Ala Lys Ile
        355                 360                 365

Phe Glu Ile Gln Thr Met Pro Asn Gly Lys Thr Arg Thr Ser Leu Lys
    370                 375                 380

Thr Met Ser Arg Arg Lys Leu Ser Gln Gln Lys Glu Lys Lys Ala Thr
385                 390                 395                 400

Gln Met Leu Ala Ile Val Leu Gly Val Ile Ile Ile Cys Trp Leu Pro
                405                 410                 415

Phe Phe Ile Thr His Ile Leu Asn Ile His Cys Asp Cys Asn Ile Pro
            420                 425                 430

Pro Val Leu Tyr Ser Ala Phe Thr Trp Leu Gly Tyr Val Asn Ser Ala
        435                 440                 445

Val Asn Pro Ile Ile Tyr Thr Thr Phe Asn Ile Glu Phe Arg Lys Ala
    450                 455                 460

Phe Leu Lys Ile Leu His Cys
465                 470
```

```
<210>  27
<211>  471
<212>  PRT
<213>  Unknown

<220>
<223>  Artificial sequence

<400>  27
```

```
Met Tyr Pro Tyr Asp Val Pro Asp Tyr Ala Tyr Pro Tyr Asp Val Pro
1               5               10              15

Asp Tyr Ala Tyr Pro Tyr Asp Val Pro Asp Tyr Ala Asp Asp Pro Leu
            20              25              30

Asn Leu Ser Trp Tyr Asp Asp Asp Leu Glu Arg Gln Asn Trp Ser Arg
            35              40              45

Pro Phe Asn Gly Ser Asp Gly Lys Ala Asp Arg Pro His Tyr Asn Tyr
        50              55              60

Tyr Ala Thr Leu Leu Thr Leu Leu Ile Ala Val Ile Val Phe Gly Asn
65              70              75              80

Val Leu Val Cys Met Ala Val Ser Arg Glu Lys Ala Leu Gln Thr Thr
                85              90              95

Thr Asn Tyr Leu Ile Val Ser Leu Ala Val Ala Asp Leu Leu Val Ala
            100             105             110

Thr Leu Val Met Pro Trp Val Val Tyr Leu Glu Val Val Gly Glu Trp
        115             120             125

Lys Phe Ser Arg Ile His Cys Asp Ile Phe Val Thr Leu Asp Val Met
        130             135             140

Met Cys Thr Ala Ser Ile Leu Asn Leu Cys Ala Ile Ser Ile Asp Arg
145             150             155             160

Tyr Thr Ala Val Ala Met Pro Met Leu Tyr Asn Thr Arg Tyr Ser Ser
                165             170             175

Lys Arg Arg Val Thr Val Met Ile Ser Ile Val Trp Val Leu Ser Phe
            180             185             190

Thr Ile Ser Cys Pro Leu Leu Phe Gly Leu Asn Asn Ala Asp Gln Asn
        195             200             205

Glu Cys Ile Ile Ala Asn Pro Ala Phe Val Val Tyr Ser Ser Ile Val
    210             215             220

Ser Phe Tyr Val Pro Phe Ile Val Met Leu Leu Val Tyr Ile Lys Ile
225             230             235             240

Tyr Ile Val Leu Arg Arg Arg Arg Lys Arg Val Asn Thr Lys Arg Ser
            245             250             255
```

```
Ser Arg Ala Phe Arg Ala His Leu Arg Ala Pro Leu Lys Gly Asn Cys
            260             265         270

Thr His Pro Glu Asp Met Lys Leu Cys Thr Val Ile Met Lys Ser Asn
        275             280             285

Gly Ser Phe Pro Val Asn Arg Arg Arg Val Glu Ala Ala Arg Arg Ala
        290             295             300

Gln Glu Leu Glu Met Glu Met Leu Ser Ser Thr Ser Pro Pro Glu Arg
305             310             315             320

Thr Arg Tyr Ser Pro Ile Pro Pro Ser His His Gln Leu Thr Leu Pro
            325             330             335

Asp Pro Ser His His Gly Leu His Ser Thr Pro Asp Ser Pro Ala Lys
            340             345             350

Pro Glu Lys Asn Gly His Ala Lys Asp His Pro Lys Ile Ala Lys Ile
            355             360             365

Phe Glu Ile Gln Thr Met Pro Asn Gly Lys Thr Arg Thr Ser Leu Lys
            370             375             380

Thr Met Ser Arg Arg Lys Leu Ser Gln Gln Lys Glu Lys Lys Ala Thr
385             390             395             400

Gln Met Leu Ala Ile Val Ile Gly Val Phe Ile Ile Cys Trp Leu Pro
                405             410             415

Phe Phe Ile Thr His Ile Leu Asn Ile His Cys Asp Cys Asn Ile Pro
            420             425             430

Pro Val Leu Tyr Ser Ala Phe Thr Trp Leu Gly Tyr Val Asn Ser Ala
            435             440             445

Val Asn Pro Ile Ile Tyr Thr Thr Phe Asn Ile Glu Phe Arg Lys Ala
            450             455             460

Phe Leu Lys Ile Leu His Cys
465             470
```

```
<210>   28
<211>   471
<212>   PRT
<213>   Unknown

<220>
```

75

<223> Artificial sequence

<400> 28

```
Met Tyr Pro Tyr Asp Val Pro Asp Tyr Ala Tyr Pro Tyr Asp Val Pro
1               5                   10                  15

Asp Tyr Ala Tyr Pro Tyr Asp Val Pro Asp Tyr Ala Asp Asp Pro Leu
            20                  25                  30

Asn Leu Ser Trp Tyr Asp Asp Asp Leu Glu Arg Gln Asn Trp Ser Arg
            35                  40                  45

Pro Phe Asn Gly Ser Asp Gly Lys Ala Asp Arg Pro His Tyr Asn Tyr
        50                  55                  60

Tyr Ala Thr Leu Leu Thr Leu Leu Ile Ala Val Ile Val Phe Gly Asn
65                  70                  75                  80

Val Leu Val Cys Met Ala Val Ser Arg Glu Lys Ala Leu Gln Thr Thr
                85                  90                  95

Thr Asn Tyr Leu Ile Val Ser Leu Ala Val Ala Asp Leu Leu Val Ala
            100                 105                 110

Thr Leu Val Met Pro Trp Val Val Tyr Leu Glu Val Val Gly Glu Trp
            115                 120                 125

Lys Phe Ser Arg Ile His Cys Asp Ile Phe Val Thr Leu Asp Val Met
    130                 135                 140

Met Cys Thr Ala Ser Ile Leu Asn Leu Cys Ala Ile Ser Ile Asp Arg
145                 150                 155                 160

Tyr Thr Ala Val Ala Met Pro Met Leu Tyr Asn Thr Arg Tyr Ser Ser
                165                 170                 175

Lys Arg Arg Val Thr Val Met Ile Ser Ile Val Trp Val Leu Ser Phe
            180                 185                 190

Thr Ile Ser Cys Pro Leu Leu Phe Gly Leu Asn Asn Ala Asp Gln Asn
            195                 200                 205

Glu Cys Ile Ile Ala Asn Pro Ala Phe Val Val Tyr Ser Ser Ile Val
    210                 215                 220

Ser Phe Tyr Val Pro Phe Ile Val Val Leu Leu Val Tyr Ile Lys Ile
225                 230                 235                 240
```

76

```
Tyr Ile Val Leu Arg Arg Arg Arg Lys Arg Val Asn Thr Lys Arg Ser
              245             250             255

Ser Arg Ala Phe Arg Ala His Leu Arg Ala Pro Leu Lys Gly Asn Cys
              260             265             270

Thr His Pro Glu Asp Met Lys Leu Cys Thr Val Ile Met Lys Ser Asn
              275             280             285

Gly Ser Phe Pro Val Asn Arg Arg Arg Val Glu Ala Ala Arg Arg Ala
              290             295             300

Gln Glu Leu Glu Met Glu Met Leu Ser Ser Thr Ser Pro Pro Glu Arg
305             310             315             320

Thr Arg Tyr Ser Pro Ile Pro Pro Ser His His Gln Leu Thr Leu Pro
              325             330             335

Asp Pro Ser His His Gly Leu His Ser Thr Pro Asp Ser Pro Ala Lys
              340             345             350

Pro Glu Lys Asn Gly His Ala Lys Asp His Pro Lys Ile Ala Lys Ile
              355             360             365

Phe Glu Ile Gln Thr Met Pro Asn Gly Lys Thr Arg Thr Ser Leu Lys
              370             375             380

Thr Met Ser Arg Arg Lys Leu Ser Gln Gln Lys Glu Lys Lys Ala Thr
385             390             395             400

Gln Met Leu Ala Ile Val Leu Gly Val Phe Ile Ile Cys Trp Leu Pro
              405             410             415

Phe Phe Ile Thr His Ile Leu Asn Ile His Cys Asp Cys Asn Ile Pro
              420             425             430

Pro Val Leu Tyr Ser Ala Phe Thr Trp Leu Gly Tyr Val Asn Ser Ala
              435             440             445

Val Asn Pro Ile Ile Tyr Thr Thr Phe Asn Ile Glu Phe Arg Lys Ala
450             455             460

Phe Leu Lys Ile Leu His Cys
465             470
```

```
<210>   29
<211>   471
```

<212>    PRT
<213>    Unknown

<220>
<223>    Artificial sequence

<400>    29

Met Tyr Pro Tyr Asp Val Pro Asp Tyr Ala Tyr Pro Tyr Asp Val Pro
1               5                   10                  15

Asp Tyr Ala Tyr Pro Tyr Asp Val Pro Asp Tyr Ala Asp Asp Pro Leu
            20                  25                  30

Asn Leu Ser Trp Tyr Asp Asp Asp Leu Glu Arg Gln Asn Trp Ser Arg
        35                  40                  45

Pro Phe Asn Gly Ser Asp Gly Lys Ala Asp Arg Pro His Tyr Asn Tyr
    50                  55                  60

Tyr Ala Thr Leu Leu Thr Leu Leu Ile Ala Val Ile Val Phe Gly Asn
65                  70                  75                  80

Val Leu Val Cys Met Ala Val Ser Arg Glu Lys Ala Leu Gln Thr Thr
                85                  90                  95

Thr Asn Tyr Leu Ile Val Ser Leu Ala Val Ala Asp Leu Leu Val Ala
            100                 105                 110

Thr Leu Val Met Pro Trp Val Val Tyr Leu Glu Val Val Gly Glu Trp
            115                 120                 125

Lys Phe Ser Arg Ile His Cys Asp Ile Phe Val Thr Leu Asp Val Met
    130                 135                 140

Met Cys Thr Ala Ser Ile Leu Asn Leu Cys Ala Ile Ser Ile Asp Arg
145                 150                 155                 160

Tyr Thr Ala Val Ala Met Pro Met Leu Tyr Asn Thr Arg Tyr Ser Ser
                165                 170                 175

Lys Arg Arg Val Thr Val Met Ile Ser Ile Val Trp Val Leu Ser Phe
            180                 185                 190

Thr Ile Ser Cys Pro Leu Leu Phe Gly Leu Asn Asn Ala Asp Gln Asn
        195                 200                 205

Glu Cys Ile Ile Ala Asn Pro Ala Phe Val Val Tyr Ser Ser Ile Val
    210                 215                 220

```
Ser Phe Tyr Val Pro Phe Ile Val Thr Leu Leu Val Leu Ile Lys Ile
225             230             235             240

Tyr Ile Val Leu Arg Arg Arg Arg Lys Arg Val Asn Thr Lys Arg Ser
            245             250             255

Ser Arg Ala Phe Arg Ala His Leu Arg Ala Pro Leu Lys Gly Asn Cys
            260             265             270

Thr His Pro Glu Asp Met Lys Leu Cys Thr Val Ile Met Lys Ser Asn
            275             280             285

Gly Ser Phe Pro Val Asn Arg Arg Arg Val Glu Ala Ala Arg Arg Ala
            290             295             300

Gln Glu Leu Glu Met Glu Met Leu Ser Ser Thr Ser Pro Pro Glu Arg
305             310             315             320

Thr Arg Tyr Ser Pro Ile Pro Pro Ser His His Gln Leu Thr Leu Pro
            325             330             335

Asp Pro Ser His His Gly Leu His Ser Thr Pro Asp Ser Pro Ala Lys
            340             345             350

Pro Glu Lys Asn Gly His Ala Lys Asp His Pro Lys Ile Ala Lys Ile
            355             360             365

Phe Glu Ile Gln Thr Met Pro Asn Gly Lys Thr Arg Thr Ser Leu Lys
            370             375             380

Thr Met Ser Arg Arg Lys Leu Ser Gln Gln Lys Glu Lys Lys Ala Thr
385             390             395             400

Gln Met Leu Ala Ile Val Leu Gly Val Phe Ile Ile Cys Trp Leu Pro
            405             410             415

Phe Phe Ile Thr His Ile Leu Asn Ile His Cys Asp Cys Asn Ile Pro
            420             425             430

Pro Val Leu Tyr Ser Ala Phe Thr Trp Leu Gly Tyr Val Asn Ser Ala
            435             440             445

Val Asn Pro Ile Ile Tyr Thr Thr Phe Asn Ile Glu Phe Arg Lys Ala
            450             455             460

Phe Leu Lys Ile Leu His Cys
465             470
```

<210>    30
<211>    471
<212>    PRT
<213>    Unknown

<220>
<223>    Artificial sequence

<400>    30

Met Tyr Pro Tyr Asp Val Pro Asp Tyr Ala Tyr Pro Tyr Asp Val Pro
1               5                   10                  15

Asp Tyr Ala Tyr Pro Tyr Asp Val Pro Asp Tyr Ala Asp Asp Pro Leu
            20                  25                  30

Asn Leu Ser Trp Tyr Asp Asp Asp Leu Glu Arg Gln Asn Trp Ser Arg
        35                  40                  45

Pro Phe Asn Gly Ser Asp Gly Lys Ala Asp Arg Pro His Tyr Asn Tyr
    50                  55                  60

Tyr Ala Thr Leu Leu Thr Leu Leu Ile Ala Val Ile Val Phe Gly Asn
65                  70                  75                  80

Val Leu Val Cys Met Ala Val Ser Arg Glu Lys Ala Leu Gln Thr Thr
                85                  90                  95

Thr Asn Tyr Leu Ile Val Ser Leu Ala Val Ala Asp Leu Leu Val Ala
            100                 105                 110

Thr Leu Val Met Pro Trp Val Val Tyr Leu Glu Val Val Gly Glu Trp
        115                 120                 125

Lys Phe Ser Arg Ile His Cys Asp Ile Phe Val Thr Leu Asp Val Met
    130                 135                 140

Met Cys Thr Ala Ser Ile Leu Asn Leu Cys Ala Ile Ser Ile Asp Arg
145                 150                 155                 160

Tyr Thr Ala Val Ala Met Pro Met Leu Tyr Asn Thr Arg Tyr Ser Ser
            165                 170                 175

Lys Arg Arg Val Thr Val Met Ile Ser Ile Val Trp Val Leu Ser Phe
        180                 185                 190

Thr Ile Ser Cys Pro Leu Leu Phe Gly Leu Asn Asn Ala Asp Gln Asn
        195                 200                 205

80

```
Glu Cys Ile Ile Ala Asn Pro Ala Phe Val Val Tyr Ser Ser Ile Val
    210             215             220

Ser Phe Tyr Val Pro Phe Ile Val Thr Leu Leu Val Tyr Ile Lys Ile
225             230             235                         240

Tyr Ile Val Leu Arg Arg Arg Arg Lys Arg Val Asn Thr Lys Arg Ser
                245             250             255

Ser Arg Ala Phe Arg Ala His Leu Arg Ala Pro Leu Lys Gly Asn Cys
            260             265             270

Thr His Pro Glu Asp Met Lys Leu Cys Thr Val Ile Met Lys Ser Asn
        275             280             285

Gly Ser Phe Pro Val Asn Arg Arg Arg Val Glu Ala Ala Arg Arg Ala
    290             295             300

Gln Glu Leu Glu Met Glu Met Leu Ser Ser Thr Ser Pro Pro Glu Arg
305             310             315                         320

Thr Arg Tyr Ser Pro Ile Pro Pro Ser His His Gln Leu Thr Leu Pro
            325             330             335

Asp Pro Ser His His Gly Leu His Ser Thr Pro Asp Ser Pro Ala Lys
            340             345             350

Pro Glu Lys Asn Gly His Ala Lys Asp His Pro Lys Ile Ala Lys Ile
    355             360             365

Phe Glu Ile Gln Thr Met Pro Asn Gly Lys Thr Arg Thr Ser Leu Lys
    370             375             380

Thr Met Ser Arg Arg Lys Leu Ser Gln Gln Lys Glu Lys Lys Ala Thr
385             390             395                         400

Gln Met Leu Ala Ile Val Leu Gly Val Phe Ile Ile Cys Trp Leu Pro
            405             410             415

Phe Phe Ile Thr His Ile Leu Asn Ile His Cys Asp Cys Asn Ile Pro
            420             425             430

Pro Val Leu Tyr Ser Ala Phe Thr Trp Leu Gly Tyr Val Asn Ser Ala
        435             440             445

Val Asn Pro Ile Ile Phe Thr Thr Phe Asn Ile Glu Phe Arg Lys Ala
    450             455             460
```

```
Phe Leu Lys Ile Leu His Cys
465                 470


<210>  31
<211>  471
<212>  PRT
<213>  Unknown

<220>
<223>  Artificial sequence

<400>  31

Met Tyr Pro Tyr Asp Val Pro Asp Tyr Ala Tyr Pro Tyr Asp Val Pro
1               5                   10                  15

Asp Tyr Ala Tyr Pro Tyr Asp Val Pro Asp Tyr Ala Asp Asp Pro Leu
            20                  25                  30

Asn Leu Ser Trp Tyr Asp Asp Asp Leu Glu Arg Gln Asn Trp Ser Arg
        35                  40                  45

Pro Phe Asn Gly Ser Asp Gly Lys Ala Asp Arg Pro His Tyr Asn Tyr
    50                  55                  60

Tyr Ala Thr Leu Leu Thr Leu Leu Ile Ala Val Ile Val Phe Gly Asn
65                  70                  75                  80

Val Leu Val Cys Met Ala Val Ser Arg Glu Lys Ala Leu Gln Thr Thr
            85                  90                  95

Thr Asn Tyr Leu Ile Val Ser Leu Ala Val Ala Asp Leu Leu Val Ala
            100                 105                 110

Thr Leu Val Met Pro Trp Val Val Tyr Leu Glu Val Val Gly Glu Trp
        115                 120                 125

Lys Phe Ser Arg Ile His Cys Asp Ile Phe Val Thr Leu Asp Val Met
    130                 135                 140

Met Cys Thr Ala Ser Ile Leu Asn Leu Cys Ala Ile Ser Ile Asp Arg
145                 150                 155                 160

Tyr Thr Ala Val Ala Met Pro Met Leu Tyr Asn Thr Arg Tyr Ser Ser
            165                 170                 175

Lys Arg Arg Val Thr Val Met Ile Ser Ile Val Trp Val Leu Ser Phe
            180                 185                 190
```

```
Thr Ile Ser Cys Pro Leu Leu Phe Gly Leu Asn Asn Ala Asp Gln Asn
    195                 200             205

Glu Cys Ile Ile Ala Asn Pro Ala Phe Val Val Tyr Ser Ser Ile Val
    210                 215             220

Ser Phe Tyr Val Pro Phe Ile Val Met Leu Leu Val Tyr Ile Lys Ile
225                 230             235                 240

Tyr Ile Val Leu Arg Arg Arg Arg Lys Arg Val Asn Thr Lys Arg Ser
                245             250                 255

Ser Arg Ala Phe Arg Ala His Leu Arg Ala Pro Leu Lys Gly Asn Cys
            260             265             270

Thr His Pro Glu Asp Met Lys Leu Cys Thr Val Ile Met Lys Ser Asn
        275             280             285

Gly Ser Phe Pro Val Asn Arg Arg Arg Val Glu Ala Ala Arg Arg Ala
    290             295             300

Gln Glu Leu Glu Met Glu Met Leu Ser Ser Thr Ser Pro Pro Glu Arg
305             310             315                 320

Thr Arg Tyr Ser Pro Ile Pro Pro Ser His His Gln Leu Thr Leu Pro
            325             330             335

Asp Pro Ser His His Gly Leu His Ser Thr Pro Asp Ser Pro Ala Lys
        340             345             350

Pro Glu Lys Asn Gly His Ala Lys Asp His Pro Lys Ile Ala Lys Ile
    355             360             365

Phe Glu Ile Gln Thr Met Pro Asn Gly Lys Thr Arg Thr Ser Leu Lys
    370             375             380

Thr Met Ser Arg Arg Lys Leu Ser Gln Gln Lys Glu Lys Lys Ala Thr
385             390             395                 400

Gln Met Leu Ala Ile Val Leu Gly Val Phe Ile Ile Cys Trp Leu Pro
            405             410             415

Phe Phe Ile Thr His Ile Leu Asn Ile His Cys Asp Cys Asn Ile Pro
        420             425             430

Pro Val Leu Tyr Ser Ala Phe Thr Trp Leu Gly Tyr Val Asn Ser Ala
    435             440             445
```

```
Val Asn Pro Ile Ile Tyr Thr Thr Phe Asn Ile Glu Phe Arg Lys Ala
    450                 455                 460

Phe Leu Lys Ile Leu His Cys
465                 470


<210>   32
<211>   471
<212>   PRT
<213>   Unknown

<220>
<223>   Artificial sequence

<400>   32

Met Tyr Pro Tyr Asp Val Pro Asp Tyr Ala Tyr Pro Tyr Asp Val Pro
1               5               10                  15

Asp Tyr Ala Tyr Pro Tyr Asp Val Pro Asp Tyr Ala Asp Asp Pro Leu
            20                  25                  30

Asn Leu Ser Trp Tyr Asp Asp Asp Leu Glu Arg Gln Asn Trp Ser Arg
            35                  40                  45

Pro Phe Asn Gly Ser Asp Gly Lys Ala Asp Arg Pro His Tyr Asn Tyr
        50                  55                  60

Tyr Ala Thr Leu Leu Thr Leu Leu Ile Ala Val Ile Val Phe Gly Asn
65                  70                  75                  80

Val Leu Val Cys Met Ala Val Ser Arg Glu Lys Ala Leu Gln Thr Thr
                85                  90                  95

Thr Asn Tyr Leu Ile Val Ser Leu Ala Val Ala Asp Leu Leu Val Ala
            100                 105                 110

Thr Leu Val Met Pro Trp Val Val Tyr Leu Glu Val Val Gly Glu Trp
            115                 120                 125

Lys Phe Ser Arg Ile His Cys Asp Ile Phe Val Thr Leu Asp Val Met
        130                 135                 140

Met Cys Thr Ala Ser Ile Leu Asn Leu Cys Ala Ile Ser Ile Asp Arg
145                 150                 155                 160

Tyr Thr Ala Val Ala Met Pro Met Leu Tyr Asn Thr Arg Tyr Ser Ser
                165                 170                 175
```

84

Lys Arg Arg Val Thr Val Met Ile Ser Ile Val Trp Val Leu Ser Phe
                180                 185                 190

Thr Ile Ser Cys Pro Leu Leu Phe Gly Leu Asn Asn Ala Asp Gln Asn
                195                 200                 205

Glu Cys Ile Ile Ala Asn Pro Ala Phe Val Val Tyr Ser Ser Ile Val
            210                 215                 220

Ser Phe Tyr Val Pro Phe Ile Val Thr Leu Leu Val Tyr Ile Lys Ile
225                 230                 235                 240

Tyr Ile Val Leu Arg Arg Arg Arg Lys Arg Val Asn Thr Lys Arg Ser
                245                 250                 255

Ser Arg Ala Phe Arg Ala His Leu Arg Ala Pro Leu Lys Gly Asn Cys
                260                 265                 270

Thr His Pro Glu Asp Met Lys Leu Cys Thr Val Ile Met Lys Ser Asn
                275                 280                 285

Gly Ser Phe Pro Val Asn Arg Arg Arg Val Glu Ala Ala Arg Arg Ala
        290                 295                 300

Gln Glu Leu Glu Met Glu Met Leu Ser Ser Thr Ser Pro Pro Glu Arg
305                 310                 315                 320

Thr Arg Tyr Ser Pro Ile Pro Pro Ser His His Gln Leu Thr Leu Pro
                325                 330                 335

Asp Pro Ser His His Gly Leu His Ser Thr Pro Asp Ser Pro Ala Lys
                340                 345                 350

Pro Glu Lys Asn Gly His Ala Lys Asp His Pro Lys Ile Ala Lys Ile
                355                 360                 365

Phe Glu Ile Gln Thr Met Pro Asn Gly Lys Thr Arg Thr Ser Leu Lys
        370                 375                 380

Thr Met Ser Arg Arg Lys Leu Ser Gln Gln Lys Glu Lys Lys Ala Thr
385                 390                 395                 400

Gln Met Leu Ala Ile Val Leu Gly Val Phe Ile Ile Cys Trp Leu Pro
                405                 410                 415

Phe Phe Ile Thr His Ile Leu Asn Ile His Cys Asp Cys Asn Ile Pro
                420                 425                 430

```
Pro Val Leu Tyr Ser Ala Phe Thr Trp Leu Gly Tyr Val Asn Ser Ala
        435                 440                 445


Val Asn Pro Ile Ile Tyr Thr Thr Phe Asn Ile Glu Phe Arg Lys Ala
        450                 455                 460


Phe Leu Lys Ile Leu His Cys
465                 470
```

## Description

### Technical Field

The present invention relates to a method for generating variants of a protein. The invention also concern a computer implemented program to carry out said method. The invention further concerns variant of a protein obtained by the method according to the present invention and polynucleotide encoding said variant.

### Background of the art

It is known that membrane receptors sense extracellular stimuli and transduce these signals into intracellular signaling responses. Subtle differences in protein sequence often give rise to profound changes in signaling response with no obvious connection to their structure.

Allostery is a fundamental property that enables long-range communication between distant sites on a molecule. Allostery is known either for protein membrane receptor or soluble protein. It is at the origin of a large diversity of regulatory mechanisms of biological molecular functions but its biophysical underpinnings remain poorly understood. Allosteric communications are thought to be primarily mediated by intraprotein networks of coupled residues (i.e. allosteric residues) physically connecting extracellular and intracellular regions of the protein. Long-range structural coupling can promote the effective communication between distant protein sites through the propagation of even small changes in local structure and dynamics.

These observations suggest that the residue couplings that regulate the intracellular responses to extracellular stimuli largely depend on amino-acid sequence details and fine protein structure and dynamic properties. Hence, predicting how protein sequences and structures encode specific allosteric responses remains particularly challenging.

Better understanding and engineering allostery in protein would greatly benefit not only the study of protein structure, function, and pharmacology but also the design of novel biosensing protein for synthetic and cell biology applications, for instance membrane receptors.

Existing methodologies structural biologists have developed are based on empirical approaches for screening stabilized mutants to facilitate structure determination. However, studying stabilized mutants, for instance thermostabilized mutants, present serious drawback notably because they frequently fail to exhibit the ligand induced signal transduction response associated with the wild type protein.

Therefore, there is a need for a methodology to facilitate protein variant engineering and studying.

### Summary of the invention

The above problems are solved at least partially by the present invention.

The invention concerns a method for generating variants of a protein based on a native protein regulated by allosteric pathway, the method comprising:

- i) providing 3D structures of the native protein, said 3D structures comprising protein active conformation and protein inactive conformation for both ligand free and ligand bound states;

- ii) identifying at least one pair of coupled allosteric sites within the amino acid sequence of the native protein, each allosteric site being named microswitch, each microswitch consisting in one amino acid involved in regulating a signal transduced by the native protein,

- iii) generating *in silico* mutations of said identified microswitch to generate a pool of variants whose amino acid sequences comprise at least one mutation compared to the native sequence,

- iv) computing at least one score reflecting

    - iv)a) a variation in allosteric coupling for each variant compared to the allosteric coupling of the native protein, said variation being named ΔG-coupling; and/or

    - iv)b) a variation in the relative stability of each conformation for each variant, said variation being named ΔG-stability;

- v) predicting the activity of each variant compared to the native protein based on the computed score ΔG-coupling or ΔG-stability, namely

- v)a) a change in ligand-induced activity of the variant compared to the native protein, said change being calculated by

$$\Delta L \text{ activity} = (\Delta G \text{ coupling})AL - (\Delta G \text{ coupling})IL;$$

and/or

- v)b) a change in ligand free activity of the variant compared to the native protein, said change being calculated by

$$\Delta C \text{ activity} = (\Delta G \text{ stability})A - (\Delta G \text{ stability})I$$

wherein for the ligand free state conformations, variant protein active and inactive conformations are denoted respectively A and I;

whereas for the ligand bound state conformations, variant protein active and inactive conformations are denoted respectively AL and IL.

A protein regulated by allosteric pathways, for instance a membrane receptor or a soluble protein, can adopt either an active conformation or an inactive conformation for both ligand free and ligand bound states. The switch between active and inactive conformation involves smaller scale movements of individual amino acid named herein as microswitch. A microswitch corresponds to one amino-acid at one specific position. Since, there are 20 possible amino-acids, the present invention can select 20 different possible microswitches at a given site.

Additionally, multiple microswitches at several sites can be designed simultaneously. Alternatively, the present invention also concerns combinations of microswitches comprising several single amino acids.

Advantageously, one microswitch is coupled to another microswitch pairwise. Alternatively, one microswitch can be paired with more than one microswitch.

The present invention allows generating variants of a protein by selective mutation of microswitches, preferably of at least one pair of coupled microswitch.

One aim of the invention is to select variant with microswitch that shifts the stability or the structural coupling of specific protein conformations.

For example, variants with increased constitutive activity, i.e. activity in ligand free state, namely stability microswitch, can be engineered by introducing the appropriate mutation of the identified microswitch providing an increased stability to the engineered variant. The point is to make more stabilizing contacts in the active versus the inactive ligand-free conformations. Advantageously, point amino acid mutation on stability microswitch allows to modulate the fraction of time the protein spends in each state.

Alternatively, variants with enhanced signaling response to ligand, namely allosteric microswitch, preferably agonist ligand, can be engineered by introducing the appropriate mutation of the identified microswitch to increase ligand/protein binding and/or signal transduction, for instance ligand/receptor. Advantageously, point amino acid mutation allows to modulate selectively the responses to ligands by enhancing or decreasing the protein allosteric sensing properties.

For allosteric microswitch, a score named $\Delta G$-coupling is computed. In one embodiment, $\Delta G$-coupling is calculated from the dynamics correlation between identified microswitch using an elastic model of the protein, for instance

$$-RT\log (\alpha_x / \alpha_y) = (G^C_{A,Lb} - G^C_{I,Lb})_x - (G^C_{A,Lb} - G^C_{I,Lb})_y = \Delta G^C_x - \Delta G^C_y = \Delta\Delta G$$

With each $G^c$ = Sum (i=1; j>i; i, j<= total nb of microswitches) (Correl_dyn (i, j)). Correl_dyn is the dynamic correlation between microswitches i and j calculated using an elastic network model of the protein.

A change in ligand-induced activity of the variant compared to the native protein is determined by comparison between the $\Delta L$ coupling of the variant compared to the one of the native protein. The $\Delta L$ activity of the variant is calculated by

- $\Delta L$ activity = ($\Delta G$ coupling)AL - ($\Delta G$ coupling)IL

wherein variant protein active and inactive conformations are denoted respectively AL, IL for ligand bound states.

For allosteric microswitch, if $\Delta L$ activity of the variant is superior to $\Delta L$ activity of the native protein, it means the variant exhibits an increased sensitivity to ligand binding compared to the native protein. If ΔL activity of the variant is inferior to ΔL activity of the native protein, it means the variant exhibits a decreased sensitivity to ligand binding compared to the native protein.

For stability microswitch, a score named ΔG-stability is computed. ΔG-stability is computed by the sum of all interactions between the residues of the protein in a specific conformation and in absence of ligand (i.e. the total free energy of the system). In other words, the ΔG-stability is computed from the free energy difference between each conformation in the absence of ligand.

A change in ligand-free activity of the variant compared to the native protein is determined by comparison between the ΔL stability of the variant compared to the one of the native protein. The ΔL activity of the variant is calculated by

- ΔL activity = (ΔG stability)A - (ΔG stability)I

- wherein variant protein active and inactive conformations are denoted A, I for respectively the ligand free and ligand states, and AL, IL for the ligand free and ligand bound states.

For stability microswitch, if ΔL activity of the variant is superior to ΔL activity of the native protein, it means the variant exhibits an increased stability compared to the native protein when bound to the ligand. If ΔL activity of the variant is inferior to ΔL activity of the native protein, it means the variant exhibits a decreased stability compared to the native protein when bound to the ligand.

In one embodiment, the prediction of the activity of the variant is based on a fitness function defined by:

$$F_{variant} = \Delta L \text{ activity (stability)} + \Delta C \text{ activity (coupling)}$$

The stability and coupling components can be calculated and studied independently because they correspond to two distinct allosteric properties. However, it is advantageous to use both stability and coupling component to fully describe any allosteric molecular system.

In an embodiment, the variation of allosteric coupling of said variant is chosen among :

- a) an increased constitutive activity for the active ligand free conformation A versus the inactive ligand free conformation I;

- b) an enhanced signaling response for the active ligand bound conformation AL versus the inactive ligand bound conformation IL.

Advantages of situation a (above):

- 1) should ease the structural, biophysical and pharmacological characterization of protein active states that are usually transient and unstable, on other words very difficult to isolate and characterize, for instance membrane receptor;

- 2) should facilitate the screening and identification of strong inhibitors that downregulated protein activities, for instance membrane receptor. Pharmaceutical industries usually seek such compounds using native protein. However, because native protein have very low intrinsic activities, inhibitors are very hard to find using such approach. Constitutively activated protein variants would provide well behaved targets with a large window of detection of potent inhibitors.

Advantages of situation b (above):

- 1) new classes of biosensors for synthetic biology, drug detection can be designed to virtually any ligand that has a propensity to bind to the protein, for instance membrane receptor;

- 2) new classes of protein therapeutics can be designed that enhance the sensitivity to a particular stimulus (e.g. for instance membrane receptor such as a dopamine receptor with enhanced sensitivity to dopamine could be used in gene therapy application for Parkinson disease patients and enable better motor control despite lower levels of dopamine in the brain);

- 3) new classes of protein therapeutics can be designed that redirect a particular extracellular signal to a intracellular activities that enhance immune responses (e.g. for instance membrane receptor such as a chimeric receptor can be designed that redirects an immune-suppressive signal from the tumor microenvironment into an activation signal inside a T-cell for enhancing cancer immunotherapies).

In one embodiment, said microswitch is identified by molecular dynamic simulations, for instance long time scale molecular dynamics. Molecular dynamic simulations are for instance described in Battacharya et al, Biophysical Journal, July 2014, 107(2), 422-434. As described in Battacharya *et al*, pairs of residues with high dynamic correlations or Mutual Information located on either binding sites (i.e. extracellular and intracellular) of the receptor are identified from the simulation trajectories. Pathways linking these sites through non-covalent interactions with other residues are identified that maximize the mutual information between all the connected residues in the pathway. For instance, allosteric microswitches are defined as the residues involved in multiple pathways.

In an embodiment, the 3D structures of the native protein is generated by homology modeling based on at least one homolog protein. Thus, the present invention can be applied to any protein with available homolog protein. The present invention is not limited to protein for which 3D data structure are available. For instance, so far, less than 5% of all GPCRs have been structurally characterized. Using homology modeling enables to expand the structural coverage and provide reliable structural models for close to 40% of all GPCRs.

In one embodiment, said in silico mutations process is based on random mutagenesis using genetic algorithm. The Genetic algorithm (GA) evolves an initial population of protein sequences to optimize its fitness over multiple generations using two genetic operators: point mutagenesis and cross over recombination between 2 sequences. For a given population of sequences, the GA will calculate the fitness for each sequence. Then, it will create a new population of sequences defining the subsequent generation by preferentially selecting and modifying a subset of the current sequences that have the highest fitness. The probabilities for point mutagenesis and cross over recombination by the GA are defined by the user beforehand.

In one embodiment, the protein is chosen among protein membrane receptor or soluble protein. A protein membrane receptor, i.e. membrane receptor, is defined as protein receptor embedded in a cellular membrane. A soluble protein can also bind a ligand, but the soluble protein is not embedded in the cellular membrane.

In an embodiment, said receptor regulated by allosteric pathway is chosen among GPCR. The method could be applied to other families of multi-pass and single-pass receptors including Cytokine, Tyrosine kinases but also transporters, channels.

In one embodiment, the method further comprises a validation step for testing, preferably *in vitro* and/or *in cellulo* testing, preferably *in vitro*, preferably *in cellulo*, the predicted activity of the selected variant. It allows to improve the success rate of computational techniques. For instance, the present invention achieves about 80% success rate. A validation step helps to identify and rank the designed protein based on their measured activities. The validation step could also help increasing the success rate by incorporating the result of the validation step to proofread or improve the method according to the present invention, for instance the generation of *in silico* mutation.

The feedback from the experiments (i.e. failures, successes) is advantageous as it enables to optimize the computational approach and parameters guiding the calculations.

In an embodiment, the method is arranged for generating a variant of protein with an improved parameter for the ligand compared to the one of said ligand with the native protein, said parameter being chosen among sensitivity (defined as the capacity of the ligand to activate the protein), selectivity, affinity, in particular sensitivity.

In one embodiment, the method is arranged for generating a variant of protein designed for interacting with a ligand distinct from or identical to the ligand interacting with the native protein. The present invention enables the design of protein (for instance receptor or biosensor) with fine-tuned properties to a large diversity of ligands without having to design protein (for instance receptor or biosensor) from scratch which is very challenging. By rationally reprogramming the function of existing protein (for instance receptor or biosensor) through a minimal number of mutations, the present invention achieves high success rate and efficiency and can be applied to design protein (for instance receptor or biosensor) for a large variety of ligands and signals.

The invention also concerns a computer implemented program comprising instructions which, when the program is executed by a computer, cause the computer to carry out the method according to the present invention.

The invention further relates to a data processing apparatus comprising means for carrying out the method according to the present invention.

The particular advantages of the data processing apparatus and of the computer implemented program are similar to the ones of the method of the invention and will thus not be repeated here.

The invention further relates to a protein, or an active fragment or variant thereof, obtained by the method according to the present invention, wherein the sequence of said protein, or active fragment or variant thereof, comprises at least one mutation in the microswitch region.

In an embodiment, the ligand sensing and/or signaling response is modified when compared to the native sequence of said protein.

In one embodiment, the ligand sensing and/or signaling response is enhanced or decreased when compared to the native sequence of said protein.

The invention also relates to a polynucleotide encoding a protein, or an active fragment or analog thereof, according to the present invention.

A "fragment" of a protein, peptide or polypeptide of the invention refers to a sequence containing less amino acids in length than the protein, peptide or polypeptide of the invention. This sequence can be used as long as it exhibits the same properties, i.e. is biologically active, as the native sequence from which it derives.

The term "analog" refers to a protein, peptide or polypeptide of the invention having an amino acid sequence that differ to some extent from a native sequence peptide, that is an amino acid sequence that varies from the native sequence by amino acid substitutions, whereby one or more amino acids are substituted by another with same characteristics and conformational roles. The amino acid sequence analogs possess substitutions, deletions, and/or insertions at certain positions within the amino acid sequence of the native amino acid sequence. Substitutions can be conservative or non-conservative. Conservative amino acid substitutions are known in the art, and include amino acid substitutions in which one amino acid having certain physical and/or chemical properties is exchanged for another amino acid that has the same chemical or physical properties. For instance, the conservative amino acid substitution may be an acidic amino acid substituted for another acidic amino acid (e.g., Asp or Glu), an amino acid with a nonpolar side chain substituted for another amino acid with a nonpolar side chain (e.g., Ala, Gly, Val, Ile, Leu, Met, Phe, Pro, Trp, Val, etc.), a basic amino acid substituted for another basic amino acid (Lys, Arg, etc.), an amino acid with a polar side chain substituted for another amino acid with a polar side chain (Asn, Cys, Gln, Ser, Thr, Tyr, etc.), etc. Preferably, the substitutions, deletions, and/or insertions at certain positions within the amino acid sequence of the amino acid sequence occur in a region that is different from the microswitch region.

In general, the sequences of such analogs will have a high degree of sequence homology to the reference sequence, e.g., sequence homology of more than 50%, generally more than 60%-70%, even more particularly 80%-85% or more, such as at least 90%-95% or more, when the two sequences are aligned.

Both the analog and fragment of the protein of the invention can include synthetic, non-standard and/or naturally-occurring amino acid sequences (including D-forms and/or retro-inverso isomers) derivable from the naturally occurring amino acid sequence of the protein of the invention. By way of example, the replacement amino acid may be a basic non-standard amino acid, (e.g. L-Ornithine, L-2-amino-3-guanidinopropionic acid, or D-isomers of Lysine, Arginine and Ornithine). Methods for introducing non-standard amino acids into proteins are known in the art, and include recombinant protein synthesis using E. coli auxotrophic expression hosts.

Non-naturally occurring amino acids include, without limitation, trans-3-methylproline, 2,4-methano-proline, cis-4-hydroxyproline, trans-4-hydroxy-proline, N-methylglycine, allo-threonine, methyl-threonine, hydroxy-ethylcysteine, hydroxyethylhomo-cysteine, nitro-glutamine, homoglutamine, pipecolic acid, tert-leucine, norvaline, 2-azaphenylalanine, 3-azaphenyl-alanine, 4-azaphenyl-alanine, and 4-fluorophenylalanine. Several methods are known in the art for incorporating non-naturally occurring amino acid residues into proteins.

The present invention also contemplates one or more polynucleotide(s) encoding a protein, or an active fragment or analog thereof, of the invention.

The present invention also contemplates a gene delivery vector or expression vector or gene therapy vector, preferably in the form of a plasmid or a vector, which comprises one or more polynucleotide(s) encoding a protein, or an active fragment or analog thereof of the invention.

As used herein, a "vector" is capable of transferring nucleic acid sequences to target cells (e.g., viral vectors, non-viral vectors, particulate carriers, and liposomes). The terms "expression vector", "gene delivery vector" and "gene therapy vector" refer to any vector that is effective to incorporate and express one or more nucleic acid(s), in a cell, preferably under the regulation of a promoter. A cloning or expression vector may comprise additional elements, for example, regulatory and/or post-transcriptional regulatory elements in addition to a promoter. The promoter can be inducible and/or cell type-specific for specific expression in a tissue (e.g. neurons such as dopaminergic or serotoninergic neurons, glial cells, ...).

Suitable vectors include derivatives of SV40 and known bacterial plasmids, e. g., E. coli plasmids col EI, pCRI, pBR322, pLive, pMB9 and their derivatives, plasmids such as RP4; phage DNAs, e. g., the numerous derivatives of phage λ, e. g., NM989, and other phage DNA, e. g., MI 3 and filamentous single stranded phage DNA; yeast plasmids such as the 2μ plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like. Various viral vectors are used for delivering nucleic acid to cells in vitro or in vivo. Non-limiting examples are vectors based on Herpes Viruses, Pox-viruses, Adeno-associated virus, Lentivirus, and others. In principle, all of them are suited to deliver the expression cassette comprising one or more polynucleotide(s) encoding the peptides of the invention, variants or fragments thereof and a promoter, optionally an inducible and/or cell type-specific promoter.

Also contemplated in the present invention is a host cell comprising a plasmid or vector of the invention or one or more nucleic acid(s) encoding the peptides of the invention, analogs or fragments thereof. The host cell can be any prokaryotic or eukaryotic cell, preferably the host cell is a eukaryotic cell, most preferably the host cell is a mammalian cell. Even more preferably, the host cell is a human neuronal or glial cell.

In one embodiment, the invention further provides pharmaceutical compositions comprising a therapeutically effective amount of i) a plasmid or a vector of the invention, or iii) a host cell of the invention, and a pharmaceutically acceptable excipient, diluent, carrier, salt and/or additive.

Alternatively, the vector is a gene delivery vector for site-directed mutagenesis, preferably a viral vector issued for delivering a gene editing system (CRISP, TALEN, etc...) comprising i) at least one sgRNA, or crRNA and tracrRNA, targeting the genomic sequence (target DNA) encoding the protein of interest, and ii) and a structure-guided endonuclease such as an RNA-guided endonuclease. Any suitable naturally occurring, or engineered, RNA-guided endonuclease can be employed as long as it is effective for specifically binding a target DNA of the invention and it may be selected from the non-limiting group comprising Cas9, Cpf1, and FEN-1. Preferably, the RNA-guided endonuclease is Cas9.

In a further embodiment, the invention provides a method of treating and/or preventing a disease such as e.g. a neurodegenerative disease or condition, or an associated symptom, in a subject in need thereof, the method comprising administering to said subject a pharmaceutical composition of the invention.

In one embodiment, the protein of the invention is selected from the group comprising from SEQ ID No. 1, SEQ ID No 2, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 29, SEQ ID No. 30, or active fragment or analog thereof, or any combination thereof.

Preferably, the protein of the invention, or active fragment or analog thereof, is characterized in that the ligand sensing and/or signaling response is modified when compared to the native sequence of said protein, or active fragment or analog thereof.

Most preferably, the ligand sensing and/or signaling response is enhanced or decreased when compared to the native sequence of said protein, or active fragment or analog thereof.

The embodiments described for the method also apply to the data processing and computer implemented program according to the present invention mutatis mutandis.

Further particular advantages and features of the invention will become more apparent from the following non-limitative description of at least one embodiment of the invention.

## Detailed description of the invention

The present detailed description is intended to illustrate the invention in a non-limitative manner since any feature of an embodiment may be combined with any other feature of a different embodiment in an advantageous manner.

In the following example, the present invention is applied on GPCR to generate variants of GPCR but the invention is not limited to this class of receptor. The present invention can also be applied to other class of protein receptor.

GPCRs are prime examples of signal transduction across biological membranes. These receptors have evolved to activate several key intracellular pathways and physiological processes in response to a large diversity of extracellular stimuli. GPCR dysfunctions including misfolding, stability loss, and altered signaling activity are often associated with serious diseases. Furthermore, drugs triggering severe side-effects have been found to activate undesired signaling pathways. Hence, the mapping of receptor stability and signal transduction pathways determinants would greatly enhance the development of effective therapies and personalized medicine approaches but has remained very challenging.

In the present example, the point was to generate (in other words engineer) dopamine D2 receptor variants.

### 3D structures:

In the present example, the 3D structures of ligand free and ligand bound states were engineered by homology modeling.

For the ligand free 3D structures, the closest structurally characterized homologs available were used as templates to model the inactive and active state. The inactive state conformations were modeled starting from the X-ray structure of the antagonist bound dopamine D3 receptor (DRD3, PDBID: 3PBL) sharing 48% sequence identity with D2. The active state conformations were modeled from the active state GPCR structures of two distant homologs: opsin (23% sequence identity with D2) bound to Gt (PDBID: 3CAP, 3DQB) and beta-2 adrenergic receptor (26% sequence identity with D2) in complex with ligand agonist and heterotrimeric Gs (PDBID: 3SN6). Models of D2 inactive and active states were generated without bound ligand or G protein using the homology mode of RosettaMembrane.

From the above-mentioned simulations, ensembles of low energy models were clustered and centers of the most populated clusters were selected as templates for each ligand-free state in the design calculations of stability microswitches. An inactive and an active state models of D2 WT were selected therefrom.

Ligand-bound D2 models were generated by performing ligand docking simulations onto the inactive and active state ligand free D2 models obtained as described above. The ligand conformer libraries were generated using Omega (OpenEye Inc.) with default parameters from Rosetta ARLS. Ligand docking was performed using a combination of Monte Carlo moves, sidechain repacking, and gradient-based minimization with the Rosetta all-atom potential developed for protein-ligand docking. Receptor backbone and sidechain torsions along with ligand torsions, position, and orientation were optimized simultaneously.

All ligand-bound receptor models for a given state (i.e. inactive or active) were gathered together and the 5% lowest-energy models were selected and ranked based on the binding energy with the ligand. The selected ligand bound models were then clustered based on structural similarity of the bound ligand conformation. The representative model of the largest cluster was selected corresponding to the ligand bound model of the D2 receptor in a given state; i.e. ligand-bound D2 inactive and ligand-bound D2 active state conformations.

Allosteric site(s), i.e. microswitches, were defined by mapping the allosteric residues identified using Molecular Dynamics simulations performed on the homologous B2AR receptor onto the above-mentioned D2 structures.

GPCR structures are composed of 3 main regions: the extracellular ligand binding pocket, the intracellular G-protein binding domain and the "transmission" transmembrane (TM) region which connects the two binding regions and allows them to communicate. GPCRs typically switch from inactive to active state conformations upon activating agonist ligand and G-protein binding at the extracellular and intracellular domains, respectively. The structural rearrangements upon receptor activation involve large intracellular reorientations of transmembrane helices (TMH), notably TMH6 and TMH7 and smaller scale movements of individual amino-acids named microswitch across the entire TM domain.

In the present example, the TM domain of the selected ligand free and ligand bound models were scanned to identify microswitches with novel allosteric properties at the position 205. Usually, larger number of positions are designed simultaneously.

In the present example, the following amino acids were identified as microswitch:

- Threonine 205 that is in close proximity to other microswitches including Phenylalanine 202, Phenylalanine 382, Isoleucine 122.

Generation of variants

Random mutagenesis using all 20 possible amino acids at position 205 and search+selection using a Genetic algorithm were performed to optimize the following fitness function :

$$F = W_{stability} \left( \Delta E_s^A - \Delta E_s^I \right) + W_{allostery} \left( \Delta E_c^A - \Delta E_c^I \right) = W_{stability} \left( \Delta\Delta E_s \right) + W_{allostery} \left( \Delta\Delta E_c \right).$$

When the microswitch is selected for stability in the active state (A) only:
$W_{stability} = 1$; $W_{allostery} = 0$; the microswitch with the highest fitness was Isoleucine 205.

When the microswitch is selected for higher sensitivity to ligand binding only, $W_{stability} = 0$; $W_{allostery} = 1$; the microswitch with the highest fitness was Methionine 205.

Computing of the score

T205I: AG-stability or $\Delta\Delta E_s$ = 5.4; AG-coupling or $\Delta\Delta E_c$ = 0.1

T205M: $\Delta$G-stability or $\Delta\Delta E_s$ = 0.1; $\Delta$G-coupling or $\Delta\Delta E_c$ = 0.8

Prediction of the activity and in vitro confirmation of the predicted activity

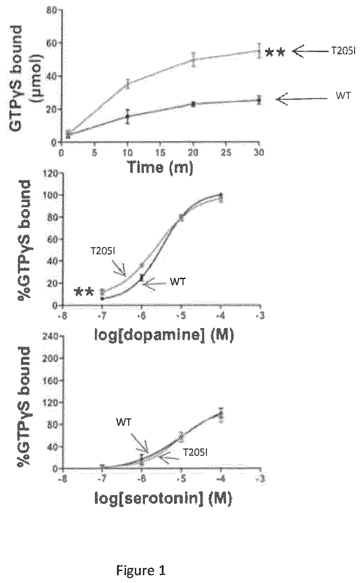

Regarding the T205I mutation, since $\Delta$G-stability/$\Delta\Delta E_s$ >0, the protein obtained by the method of the invention should have an increased constitutive activity compared to the native receptor. This prediction was confirmed by in vitro testing where this increased of constitutive activity was observed experimentally (top panel of Figure 1. Figure 1 shows the *in vitro* measurements of D2 activation. top. constitutive (time course); bottom. Agonist induced (dose response) as percent of maximal dopamine induced D2 WT activity. ** two-sided unpaired t-test: $p < 0.05$.). Therefore, the present invention allows to generate a D2 receptor stabilized in specific states, here in state A.

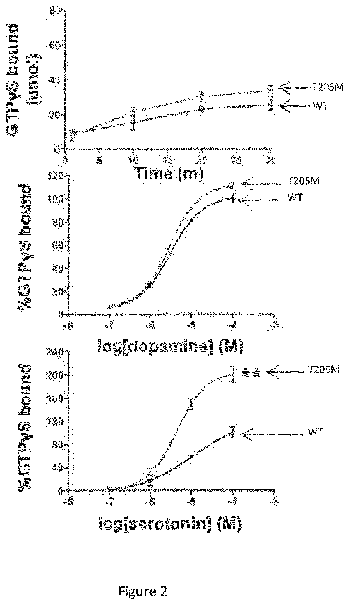

Regarding the T205M mutation, since ΔG-coupling/ΔΔEc >0, the protein obtained by the method of the invention should have a higher sensitivity for the ligand binding (ligand = dopamine and serotonin). This prediction was confirmed by in vitro testing where this increased of sensitivity was observed experimentally (bottom panel of Figure 2. Figure 2 shows the *in vitro* measurements of D2 activation. top. constitutive (time course); bottom. Agonist induced (dose response) as percent of maximal dopamine induced D2 WT activity. ** two-sided unpaired t-test: $p < 0.05$.). Therefore, the present invention allows to generate a D2 receptor with an increased sensitivity for both dopamine and serotonin ligands.

While the embodiments have been described in conjunction with a number of embodiments, it is evident that many alternatives, modifications and variations would be or are apparent to those of ordinary skill in the applicable arts. Accordingly, this disclosure is intended to embrace all such alternatives, modifications, equivalents and variations that are within the scope of this disclosure. This for example particularly the case regarding the different apparatuses which can be used.

**Sequences**

| SEQ ID No. | Name | Name / Sequence |
|---|---|---|
| 1 | C385L | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIILWLPFFIT HILNIHCDCNIPPVLYSAFTWLGYVNSAVN PIIYTTFNIEFRKAFLKILHC |
| 2 | C385V | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIIVWLPFFIT HILNIHCDCNIPPVLYSAFTWLGYVNSAVN PIIYTTFNIEFRKAFLKILHC |

(continued)

| SEQ ID No. | Name | Name / Sequence |
|---|---|---|
| 3 | F202M | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLIVSLAVADLLVATLVMPWVV<br>YLEVVGEWKFSRIHCDIFVTLDVMMCTASI<br>LNLCAISIDRYTAVAMPMLYNTRYSSKRRV<br>TVMISIVWVLSFTISCPLLFGLNNADQNEC<br>IIANPAFVVYSSIVSFYVPMIVTLLVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br>DHPKIAKIFEIQTMPNGKTRTSLKTMSRRK<br>LSQQKEKKATQMLAIVLGVFIICWLPFFIT<br>HILNIHCDCNIPPVLYSAFTWLGYVNSAVN<br>PIIYTTFNIEFRKAFLKILHC |
| 4 | F382I-C385L | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLIVSLAVADLLVATLVMPWVV<br>YLEVVGEWKFSRIHCDIFVTLDVMMCTASI<br>LNLCAISIDRYTAVAMPMLYNTRYSSKRRV<br><br>TVMISIVWVLSFTISCPLLFGLNNADQNEC<br>IIANPAFVVYSSIVSFYVPFIVTLLVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br>DHPKIAKIFEIQTMPNGKTRTSLKTMSRRK<br>LSQQKEKKATQMLAIVLGVIIILWLPFFIT<br>HILNIHCDCNIPPVLYSAFTWLGYVNSAVN<br>PIIYTTFNIEFRKAFLKILHC |
| 5 | F382I | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLIVSLAVADLLVATLVMPWVV<br>YLEVVGEWKFSRIHCDIFVTLDVMMCTASI<br>LNLCAISIDRYTAVAMPMLYNTRYSSKRRV<br>TVMISIVWVLSFTISCPLLFGLNNADQNEC<br>IIANPAFVVYSSIVSFYVPFIVTLLVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br>DHPKIAKIFEIQTMPNGKTRTSLKTMSRRK<br>LSQQKEKKATQMLAIVLGVIIICWLPFFIT<br>HILNIHCDCNIPPVLYSAFTWLGYVNSAVN<br>PIIYTTFNIEFRKAFLKILHC |

(continued)

| SEQ ID No. | Name | Name / Sequence |
|---|---|---|
| 6 | F382Y | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLIVSLAVADLLVATLVMPWVV<br>YLEVVGEWKFSRIHCDIFVTLDVMMCTASI<br>LNLCAISIDRYTAVAMPMLYNTRYSSKRRV<br>TVMISIVWVLSFTISCPLLFGLNNADQNEC<br>IIANPAFVVYSSIVSFYVPFIVTLLVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br>DHPKIAKIFEIQTMPNGKTRTSLKTMSRRK<br>LSQQKEKKATQMLAIVLGVYIICWLPFFIT<br>HILNIHCDCNIPPVLYSAFTWLGYVNSAVN<br>PIIYTTFNIEFRKAFLKILH |
| 7 | I122F | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLIVSLAVADLLVATLVMPWVV<br>YLEVVGEWKFSRIHCDIFVTLDVMMCTASF<br>LNLCAISIDRYTAVAMPMLYNTRYSSKRRV<br>TVMISIVWVLSFTISCPLLFGLNNADQNEC<br>IIANPAFVVYSSIVSFYVPFIVTLLVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br><br>DHPKIAKIFEIQTMPNGKTRTSLKTMSRRK<br>LSQQKEKKATQMLAIVLGVFIICWLPFFIT<br>HILNIHCDCNIPPVLYSAFTWLGYVNSAVN<br>PIIYTTFNIEFRKAFLKILHC |
| 8 | I122L | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLIVSLAVADLLVATLVMPWVV<br>YLEVVGEWKFSRIHCDIFVTLDVMMCTASL<br>LNLCAISIDRYTAVAMPMLYNTRYSSKRRV<br>TVMISIVWVLSFTISCPLLFGLNNADQNEC<br>IIANPAFVVYSSIVSFYVPFIVTLLVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br>DHPKIAKIFEIQTMPNGKTRTSLKTMSRRK<br>LSQQKEKKATQMLAIVLGVFIICWLPFFIT<br>HILNIHCDCNIPPVLYSAFTWLGYVNSAVN<br>PIIYTTFNIEFRKAFLKILHC |

(continued)

| SEQ ID No. | Name | Name / Sequence |
|---|---|---|
| 9 | I122T | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTAST LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIYTTFNIEFRKAFLKILHC |
| 10 | L379I_C385L | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVIGVFIILWLPFFIT HILNIHCDCNIPPVLYSAFTWLGYVNSAVN PIIYTTFNIEFRKAFLKILHC |
| 11 | L379I_F382I | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVIGVIIICWLPFFIT HILNIHCDCNIPPVLYSAFTWLGYVNSAVN PIIYTTFNIEFRKAFLKILHC |

(continued)

| SEQ ID No. | Name | Name / Sequence |
|---|---|---|
| 12 | L379I | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVIGVFIICWLPFFIT HILNIHCDCNIPPVLYSAFTWLGYVNSAVN PIIYTTFNIEFRKAFLKILHC |
| 13 | L76M | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSMAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIYTTFNIEFRKAFLKILHC |
| 14 | M90V | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVVPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIYTTFNIEFRKAFLKILHC |

(continued)

| SEQ ID No. | Name | Name / Sequence |
|---|---|---|
| 15 | D2_R1 | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIS HILNIHCDCNIPPVGYSAWTWLGYVNSAVN PIIYTTFNIEFRKAFLKILHC |
| 16 | R1+R2+R3+R4 | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLYVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPLIVIILVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQLLAIYLGLFLICWLPFFIS HILNIHCDCNIPPVGYSAWTWLGYVNSAIN PIIFATFNIEFRKAFLKILHC |
| 17 | D2_R2 | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPLIVIILVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFLICWLPFFIT HILNIHCDCNIPPVLYSAFTWLGYVNSAVN PIIYTTFNIEFRKAFLKILHC |

(continued)

| SEQ ID No. | Name | Name / Sequence |
|---|---|---|
| 18 | R2+R3 | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPLIVIILVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQLLAIYLGLFLICWLPFFIT HILNIHCDCNIPPVLYSAFTWLGYVNSAIN PIIYTTFNIEFRKAFLKILHC |
| 19 | R2+R3+R4 | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLYVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPLIVIILVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQLLAIYLGLFLICWLPFFIT HILNIHCDCNIPPVLYSAFTWLGYVNSAIN PIIFATFNIEFRKAFLKILHC |
| 20 | D2_R3 | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVILLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQLLAIYLGLFIICWLPFFIT HILNIHCDCNIPPVLYSAFTWLGYVNSAIN PIIYTTFNIEFRKAFLKILHC |
| 21 | D2_R4 | MYPYDVPDYAYPYDVPDYAYPYDVPDYADD PLNLSWYDDDLERQNWSRPFNGSDGKADRP |

(continued)

| SEQ ID No. | Name | Name / Sequence |
|---|---|---|
| | | HYNYYATLLTLLIAVIVFGNVLVCMAVSRE KALQTTTNYLYVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIFATFNIEFRKAFLKILHC |
| 22 | T205I | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVILLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIYTTFNIEFRKAFLKILHC |
| 23 | T205L | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVLLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIYTTFNIEFRKAFLKILHC |

(continued)

| SEQ ID No. | Name | Name / Sequence |
|---|---|---|
| 24 | T205M_C385L | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVMLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIILWLPFFIT HILNIHCDCNIPPVLYSAFTWLGYVNSAVN PIIYTTFNIEFRKAFLKILHC |
| 25 | T205M_C385V | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVMLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIIVWLPFFIT HILNIHCDCNIPPVLYSAFTWLGYVNSAVN PIIYTTFNIEFRKAFLKILHC |
| 26 | T205M_F382I | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVMLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIIVWLPFFIT HILNIHCDCNIPPVLYSAFTWLGYVNSAVN PIIYTTFNIEFRKAFLKILHC |

(continued)

| SEQ ID No. | Name | Name / Sequence |
|---|---|---|
| 27 | T205M_L379I | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVMLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVIGVFIICWLPFFIT HILNIHCDCNIPPVLYSAFTWLGYVNSAVN PIIYTTFNIEFRKAFLKILHC |
| 28 | T205V | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVVLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIYTTFNIEFRKAFLKILHC |
| 29 | Y209L | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVLIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIYTTFNIEFRKAFLKILHC |

(continued)

| SEQ ID No. | Name | Name / Sequence |
|---|---|---|
| 30 | Y426F | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIFTTFNIEFRKAFLKILHC |
| 31 | T205M | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV<br><br>YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVMLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIYTTFNIEFRKAFLKILHC |
| 32 | D2WT | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIYTTFNIEFRKAFLKILHC |

**Claims**

1. Method for generating variants of a protein based on a native protein regulated by allosteric pathway, the method comprising:

   - i) providing 3D structures of the native protein, said 3D structures comprising protein active conformation and inactive conformation for both ligand free and ligand bound states;
   - ii) identifying at least one pair of coupled allosteric sites within the amino acid sequence of the native protein, each allosteric site being named microswitch, each microswitch consisting in one amino acid involved in regulating a signal transduced by the native protein,
   - iii) generating *in silico* mutations of said identified microswitch to generate a pool of variants whose amino acid sequences comprise at least one mutation compared to the native sequence,
   - iv) computing at least one score reflecting

     - iv)a) a variation in allosteric coupling for each variant compared to the allosteric coupling of the native protein, said variation being named ΔG-coupling; and/or
     - iv)b) a variation in the relative stability of each conformation for each variant, said variation being named ΔG-stability;

   - v) predicting the activity of each variant compared to the native protein based on the computed score ΔG-coupling or ΔG-stability, namely

     - v)a) a change in ligand-induced activity of the variant compared to the native protein, said change being calculated by

$$\Delta L \text{ activity} = (\Delta G \text{ coupling})AL - (\Delta G \text{ coupling})IL;$$

     and/or
     - v)b) a change in ligand free activity of the variant compared to the native protein, said change being calculated by

$$\Delta C \text{ activity} = (\Delta G \text{ stability})A - (\Delta G \text{ stability})I$$

   wherein for the ligand free state conformations, variant protein active and inactive conformations are denoted respectively A and I;
   whereas for the ligand bound state conformations, variant protein active and inactive conformations are denoted respectively AL and IL.

2. Method according to claim 1, wherein the prediction of the activity of the variant is based on a fitness function defined by:

$$F_{variant} = \Delta L \text{ activity (stability)} + \Delta C \text{ activity (coupling)}$$

3. Method according to claim 1 or 2, wherein ΔG-coupling is calculated from dynamics correlations between identified microswitches using an elastic model of the protein.

4. Method according to any one of claims 1 to 3, wherein ΔG-stability is calculated from the sum of all interactions between the amino acids of the protein in a specific conformation and in absence of ligand.

5. Method according to any one of claims 1 to 4, wherein the variation of allosteric coupling of said variant is chosen among :

   - a) an increased constitutive activity for the active ligand free conformation A versus the inactive ligand free conformation I;
   - b) an enhanced signaling response for the active ligand bound conformation AL versus the inactive ligand bound conformation IL.

6. Method according to any one of claims 1 to 5, wherein the protein is chosen among membrane receptor, soluble protein, for instance GPCR, cytokine, tyrosine kinases, transporters, channels.

7. Method according to any one of claims 1 to 6 further comprising a validation step for testing, preferably *in vitro* testing, the predicted activity of a selected variant.

8. Method according to any one of claims 1 to 7, wherein the method is arranged for generating a variant of protein with an improved parameter for the ligand compared to the one of said ligand with the native protein, said parameter being chosen among selectivity, specificity, affinity, preferably sensitivity.

9. Method according to any one of claims 1 to 8, wherein the method is arranged for generating a variant of protein designed for interacting with a ligand distinct from or identical to the ligand interacting with the native protein.

10. Computer implemented program comprising instructions which, when the program is executed by a computer, cause the computer to carry out the method of any one of claims 1 to 9.

11. A protein, or an active fragment or analog thereof, obtained by the method of any one of claims 1 to 9, wherein the sequence of said variant of a protein, or active fragment thereof, comprises at least one mutation in the microswitch region.

12. The protein according to claim 11 selected from the group comprising SEQ ID No. 1, SEQ ID No 2, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 29, SEQ ID No. 30, or active fragment or analog thereof, or any combination thereof.

13. The protein, or active fragment or analog thereof, according to any one of claims 11-12, wherein the ligand sensing and/or signaling response is modified when compared to the native sequence of said protein.

14. The protein, or active fragment or analog thereof, according to any one of claim 13, wherein the ligand sensing and/or signaling response is enhanced or decreased when compared to the native sequence of said protein.

15. A polynucleotide encoding a protein, or an active fragment or analog thereof, of any one of claims 11 to 14.

Figure 1

Figure 2

Europäisches
Patentamt

European
Patent Office

Office européen
des brevets

**EUROPEAN SEARCH REPORT**

Application Number

EP 19 18 9259

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/18590 A2 (UNIV JOHNS HOPKINS [US]; BEACHY PHILIP A [US]; TAIPALE JUSSI [US]) 7 March 2002 (2002-03-07) * p.4 l.18-30, p.5 l.15, p.10 l.29-30, p.31 l.18-20, p.32 l.7-8 * | 1-10 | INV. G16B15/30 G16B15/20 C07K14/705 C07K14/72 |
| X | WO 2010/149964 A2 (HEPTARES THERAPEUTICS LTD [GB]; JAZAYERI-DEZFULY SEYED ALI [GB] ET AL.) 29 December 2010 (2010-12-29) * p.4 l.13-14, p.11 l.30-33, p.18 l.19-21, p.29 l.31-32, p.39 l.2-5, p.40 l.28-30 * | 1-10 | |
| X | WO 2014/198528 A2 (SCHERRER INST PAUL [CH]) 18 December 2014 (2014-12-18) * abstract, p.6 l.36-37, p.7 l.1-2, l.7-12, p.11, p.15 l.5-9, p.16 l.12-15 * | 1-10 | |
| A | WHITE K L ET AL: "Structural Connection between Activation Microswitch and Allosteric Sodium Site in GPCR Signaling", STRUCTURE, vol. 26, no. 2, 1 February 2018 (2018-02-01), pages 259-269.e5, XP055663000, AMSTERDAM, NL ISSN: 0969-2126, DOI: 10.1016/j.str.2017.12.013 * the whole document * | 1-10 | TECHNICAL FIELDS SEARCHED (IPC) G16B C07K |
| A | US 2019/147985 A1 (BOWMAN GREGORY R [US]) 16 May 2019 (2019-05-16) * abstract * * [0034] * | 1-10 | |

-/--

~~The present search report has been drawn up for all claims~~

| Place of search | Date of completion of the search | Examiner |
|---|---|---|
| Munich | 3 February 2020 | Werner, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

## EUROPEAN SEARCH REPORT

Application Number

EP 19 18 9259

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MAJESKE N ET AL: "Elucidating Which Pairwise Mutations Affect Protein Stability: An Exhaustive Big Data Approach", 2017 IEEE 41ST ANNUAL COMPUTER SOFTWARE AND APPLICATIONS CONFERENCE (COMPSAC), IEEE, vol. 1, 23 July 2018 (2018-07-23), pages 508-515, XP033409512, ISSN: 0730-3157, DOI: 10.1109/COMPSAC.2018.00078 ISBN: 978-1-5386-2667-2 [retrieved on 2018-06-08] * the whole document * | 1-10 | |
| A | US 2011/053261 A1 (LARIO PAULA I [CA] ET AL) 3 March 2011 (2011-03-03) * abstract * * [0088], [0091]-[0092] * | 1-10 | |

TECHNICAL FIELDS
SEARCHED      (IPC)

~~The present search report has been drawn up for all claims~~

| Place of search | Date of completion of the search | Examiner |
|---|---|---|
| Munich | 3 February 2020 | Werner, Andreas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

Application Number

EP 19 18 9259

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-10

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 19 18 9259

The Search Division considers that the present European patent application does not comply with the requirements of unity of invention and relates to several inventions or groups of inventions, namely:

1. claims: 1-10

   Method for generating variants of a protein based on a native protein regulated by allosteric pathway, the method comprising providing a 3D structure, identifying a microswitch and generating in silico mutations, from which scores on deltaG-coupling and deltaG-stability are computed, and ligand-bound and ligand-free activity is predicted; computer implemented program comprising instructions which, when the program is executed by a computer, cause the computer to carry out the above method.

   ---

2. claims: 11-15

   Protein, or an active fragment or analog thereof, wherein the sequence of said variant of a protein, or active fragment thereof, comprises at least one mutation in the microswitch region,
   preferably a protein selected from SEQ ID NO:1 to SEQ ID NO:30, and/or wherein its ligand sensing and/or signaling response is modified, more preferably enhanced or decreased, when compared with the native protein;
   Polynucleotide encoding said protein, or an active fragment or analog thereof.

   ---

## ANNEX TO THE EUROPEAN SEARCH REPORT
## ON EUROPEAN PATENT APPLICATION NO.

EP 19 18 9259

This annex lists the patent family members relating to the patent documents cited in the above-mentioned European search report.
The members are as contained in the European Patent Office EDP file on
The European Patent Office is in no way liable for these particulars which are merely given for the purpose of information.

03-02-2020

| Patent document cited in search report | | Publication date | Patent family member(s) | | Publication date |
|---|---|---|---|---|---|
| WO 0218590 | A2 | 07-03-2002 | AU | 8694501 A | 13-03-2002 |
| | | | US | 2002157119 A1 | 24-10-2002 |
| | | | US | 2009088339 A1 | 02-04-2009 |
| | | | WO | 0218590 A2 | 07-03-2002 |
| WO 2010149964 | A2 | 29-12-2010 | EP | 2445931 A2 | 02-05-2012 |
| | | | JP | 2012530690 A | 06-12-2012 |
| | | | US | 2012165507 A1 | 28-06-2012 |
| | | | WO | 2010149964 A2 | 29-12-2010 |
| WO 2014198528 | A2 | 18-12-2014 | CA | 2914831 A1 | 18-12-2014 |
| | | | CN | 105593240 A | 18-05-2016 |
| | | | EP | 3008083 A2 | 20-04-2016 |
| | | | JP | 6192818 B2 | 06-09-2017 |
| | | | JP | 2016526664 A | 05-09-2016 |
| | | | US | 2016139155 A1 | 19-05-2016 |
| | | | WO | 2014198528 A2 | 18-12-2014 |
| US 2019147985 | A1 | 16-05-2019 | US | 2019147985 A1 | 16-05-2019 |
| | | | WO | 2017192872 A1 | 09-11-2017 |
| US 2011053261 | A1 | 03-03-2011 | AU | 2009211148 A1 | 13-08-2009 |
| | | | CA | 2715043 A1 | 13-08-2009 |
| | | | EP | 2245571 A2 | 03-11-2010 |
| | | | JP | 5530367 B2 | 25-06-2014 |
| | | | JP | 2011514509 A | 06-05-2011 |
| | | | US | 2011053261 A1 | 03-03-2011 |
| | | | WO | 2009098596 A2 | 13-08-2009 |

EPO FORM P0459

For more details about this annex : see Official Journal of the European Patent Office, No. 12/82

**REFERENCES CITED IN THE DESCRIPTION**

**Non-patent literature cited in the description**

- **BATTACHARYA et al.** *Biophysical Journal,* July 2014, vol. 107 (2), 422-434